# EUROPEAN PATENT APPLICATION

(11) **EP 4 378 495 A2**
(43) Date of publication of application: **05.06.2024**
(21) Application number: 23211459.5
(22) Date of filing: 22.11.2023
(51) Int. Cl.: A61M 1/14, A61M 1/36

(54) **EXTRACORPOREAL LIFE SUPPORT SYSTEM**

(30) Priority: 02.12.2022 US 202218074165
(71) Applicant: LivaNova Deutschland GmbH, 80939 Munich (DE)
(72) Inventor: Artelsmair, Andreas, 81739 München (DE); Kohnen, Lena, 81369 München (DE); Penka, Ottmar, 81245 München (DE); Herzog, Helen, 85748 Garching bei München (DE)
(74) Representative: Grünecker Patent- und Rechtsanwälte PartG mbB

(57) **Abstract**

An extracorporeal blood treatment system may include a clamp coupled to a blood pathway extending between a patient and a reservoir, a sensor positioned along the blood pathway, and a control unit in communication with both the clamp and the sensor. The sensor is configured to sense a parameter of blood passing through the blood pathway and the sensor is configured to transmit a signal corresponding to the parameter to the control unit. The control unit is configured to receive the signal and transmit a signal to the clamp. The clamp is configured to receive the signal from the control unit and controllably adjust blood flow through the blood pathway in response to receiving the signal from the control unit.

## Description

### TECHNICAL FIELD

The present disclosure relates to an extracorporeal life support system and methods for manufacturing and/or using an extracorporeal life support system.

### BACKGROUND

Some medical procedures (e.g., medical procedures which treat cardiac or respiratory disease) may require the use of a life support system that supports cardiac and pulmonary functions by artificially supporting the heart and the lung function. In some instances, this may be carried out by an extracorporeal perfusion system. An extracorporeal perfusion system may provide both cardiac and respiratory support to a patient whose heart and lungs are unable to provide an adequate amount of gas exchange during a cardiac and pulmonary procedure. Extracorporeal perfusion works by removing blood from a patient's body to oxygenate the red blood cells while also removing carbon dioxide. The oxygenated blood is then returned to the patient.

Extracorporeal perfusion systems may include multiple devices that together form a blood recirculation loop between the patient and a blood oxygenator. For example, some extracorporeal perfusion systems may include a blood reservoir, a blood pump to power blood flow, an oxygenator to oxygenate the blood, a device to filter the blood (which may be included within the oxygenator and/or the reservoir in some systems), a heat exchanger to heat and/or cool blood (in some examples the heat exchanger may be included in the oxygenator), one or more sensors positioned at various locations along blood pathways and one or more control units. It can be appreciated that a blood pathway (e.g., tubing) may extend from the patient to the blood reservoir, then towards a blood pump, then pass through the oxygenator and close the loop by returning to the patient. Accordingly, the blood pump may assist the heart by pumping blood through the circulation loop, while the oxygenator may assist the lungs by oxygenating blood that is eventually returned to the patient.

It can be further appreciated that the amount of oxygen that can be delivered to the patient may be a function of the flow rate of the blood cycling through the circulation loop. However, there may be instances in which the flowrate of blood being taken from and returned to the patient may need to be monitored, adjusted, restricted or stopped. Therefore, it may be desirable to design an extracorporeal perfusion system which may include one or more closed-feed loops configured to monitor the flow of blood within the extracorporeal perfusion system. Extracorporeal perfusion systems including closed-feed loops configured to monitor the flow of blood within the extracorporeal perfusion system are disclosed herein.

### SUMMARY

An example extracorporeal blood treatment system may include a first clamp coupled to a first blood pathway extending between a patient and a reservoir, a first sensor positioned along the first blood pathway, and a control unit in communication with both the first clamp and the first sensor. The first sensor is configured to sense a first parameter of blood passing through the first blood pathway and the first sensor is configured to transmit a first signal corresponding to the first parameter to the control unit. The control unit is configured to receive the first signal and transmit a second signal to the first clamp. The first clamp is configured to receive the second signal from the control unit and controllably adjust blood flow through the first pathway in response to receiving the second signal from the control unit.

In addition or alternatively to any example described herein, the first sensor parameter is a first flowrate of blood passing through the first blood pathway.

In addition or alternatively to any example described herein, the first clamp is configured to decrease the first flowrate of blood flowing through the first blood pathway in response to receiving the second signal from the control unit.

In addition or alternatively to any example described herein, the first clamp is configured to increase the first flowrate of blood flowing through the first blood pathway in response to receiving the second signal from the control unit.

In addition or alternatively to any example described herein, the first sensor is directly attached to the first clamp.

In addition or alternatively to any example described herein, the first sensor is spaced away from the first clamp along the first blood pathway.

In addition or alternatively to any example described herein, the first blood pathway defines a venous pathway from the patient to the reservoir.

In addition or alternatively to any example described herein, further including a first pump in communication with the control unit.

In addition or alternatively to any example described herein, the control unit is configured to adjust a speed of the first pump based upon the first signal received from the first sensor.

In addition or alternatively to any example described herein, the first clamp is configured to automatically close to a shutdown condition in response to a shutdown signal from the control unit.

In addition or alternatively to any example described herein, the first blood pathway defines an arterial return pathway from the reservoir to the patient.

In addition or alternatively to any example described herein, further including a second sensor positioned along a second blood pathway. The second sensor is in communication with the control unit.

In addition or alternatively to any example described herein, the second sensor is configured to sense a second parameter of blood passing through the second blood pathway. The second sensor is configured to transmit a third signal corresponding to the second parameter to the control unit. The control unit is configured to receive the third signal and transmit a fourth signal to the first pump. The first pump is configured to adjust blood flow through the second blood pathway in response to receiving the fourth signal from the control unit.

In addition or alternatively to any example described herein, the second parameter is a second flowrate of the blood passing through the second blood pathway.

In addition or alternatively to any example described herein, the first blood pathway defines a venous pathway from the patient to the reservoir, and wherein the second blood pathway defines an arterial return pathway from the reservoir back to the patient.

In addition or alternatively to any example described herein, the control unit is configured to adjust a speed of the first pump based upon the third signal received from the second sensor.

In addition or alternatively to any example described herein, adjusting the speed of the first pump adjusts the second flowrate of the blood passing through the second blood pathway.

Another example extracorporeal blood treatment system includes a clamp coupled to a venous blood pathway extending between a patient and a reservoir, a fluid level sensor coupled to the reservoir, and a control unit in communication with the clamp and the level sensor. The level sensor is configured to sense a level of blood in the reservoir and transmit a first signal to the control unit that corresponds to a volume of blood in the reservoir. The control unit is configured to receive the first signal and transmit a second signal to the clamp. The clamp is configured to receive the second signal from the control unit. The clamp is configured to controllably adjust blood flow through the venous pathway in response to receiving the second signal from the control unit.

In addition or alternatively to any example described herein, a first pump positioned in the venous blood pathway, wherein the first pump is in communication with the control unit, and wherein the control unit is configured to adjust a speed of the first pump based upon the first signal received from the level sensor.

Another extracorporeal blood treatment system includes a first clamp coupled to a venous blood pathway extending between a patient and a reservoir, a first sensor positioned along the venous blood pathway, a second clamp coupled to an arterial blood pathway extending between a patient and a reservoir, a second sensor positioned along the arterial blood pathway, and a control unit in communication with the first clamp, the first sensor, the second clamp and the second sensor. The first sensor is configured to transmit a first signal to the control unit, wherein the first signal corresponds to a flowrate of blood in the venous blood pathway. The second sensor is configured to transmit a second signal to the control unit, wherein the second signal corresponds to a flowrate of blood in the arterial blood pathway. The control unit is configured to receive the first signal and the second signal and compare the first signal and the second signal. The control unit is configured to actuate the first clamp, the second clamp or both the first clamp and the second clamp in response to comparing the first signal and the second signal to controllably adjust blood flow through the venous pathway, the arterial blood pathway or both the venous pathway and the arterial blood pathway.

The above summary of some embodiments, aspects, and/or examples is not intended to describe each disclosed embodiment or every implementation of the present disclosure. The figures and detailed description which follow more particularly exemplify these embodiments.

### BRIEF DESCRIPTION OF THE DRAWINGS

The disclosure may be more completely understood in consideration of the following detailed description in connection with the accompanying drawings, in which:
FIG. 1 illustrates an example extracorporeal blood treatment system;
FIG. 2 is a schematic diagram of a computing device;
FIG. 3 illustrates another example extracorporeal blood treatment system;
FIG. 4 illustrates another example extracorporeal blood treatment system;
FIG. 5 illustrates another example extracorporeal blood treatment system;
FIG. 6 illustrates another example extracorporeal blood treatment system;
FIG. 7 illustrates another example extracorporeal blood treatment system;
FIG. 8 illustrates another example extracorporeal blood treatment system;
FIG. 9 illustrates another example extracorporeal blood treatment system;
FIG. 10 illustrates another example extracorporeal blood treatment system;
FIG. 11 illustrates another example extracorporeal blood treatment system;
FIG. 12 illustrates another example extracorporeal blood treatment system;
FIG. 13 illustrates another example extracorporeal blood treatment system;
FIG. 14 illustrates another example extracorporeal blood treatment system;
FIG. 15 illustrates another example extracorporeal blood treatment system;
FIG. 16 illustrates another example extracorporeal blood treatment system.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the spirit and scope of the disclosure.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5). As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used in connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the disclosure.

In a normal heart, blood circulates via a closed path whereby deoxygenated (venous) blood enters the right atrium via both the superior vena cava and inferior vena cava. The venous blood then passes through the right ventricle and is pumped via the pulmonary artery to the lungs, where it absorbs oxygen and releases carbon dioxide. After absorbing oxygen and releasing carbon dioxide in the lungs, the blood becomes oxygenated arterial blood. The oxygenated blood is then returned via the pulmonary veins to the left atrium and is passed to the left ventricle. The oxygenated arterial blood is then pumped through the aorta and eventually throughout the body.

It can be appreciated that if the lungs are incapable of sufficiently oxygenating blood and/or removing carbon dioxide, an oxygenator located outside the body may be used to oxygenate the blood and/or remove carbon dioxide. As discussed above, extracorporeal perfusion is a breathing and heart pumping life support system that may be utilized to support patients while medical treatments (e.g., heart surgery) are performed to treat their underlying illness. When supported via an extracorporeal perfusion system, oxygenation of the patient's blood and removal of carbon dioxide may occur outside the body.

Extracorporeal perfusion is generally performed using a heart-lung bypass system, which may be referred to as a "circuit." The circuit may include a blood flowpath exterior of the patient, such as one or more tubing pathways designed to transfer blood from a patient's body to the oxygenator and back into the patient. As described above, the oxygenator may add oxygen to the blood while also removing carbon dioxide (e.g., the oxygenator performs the function of a healthy lung).

In some examples, an extracorporeal perfusion circuit may include a blood pump, oxygenator, tubing pathways (for transfer to and from the body), sensors (e.g., flow, pressure, bubble, temperature, oxygen, carbon dioxide, etc.), a heat exchanger (to cool and/or heat the blood), a control unit, and arterial and/or venous access points for the collection of blood in the circuit. It can be appreciated that the function of the blood pump is to generate blood flow within the extracorporeal perfusion circuit (e.g., circulate blood from the patient to the oxygenator and back to the patient) and to also generate blood pressure within the patient's vascular system. The blood pump may be positioned in the tubing pathway between the patient and the oxygenator. In some extracorporeal perfusion systems a roller pump may be utilized to generate blood flow within the extracorporeal perfusion circuit. However, in other extracorporeal perfusion systems, other blood pumps, including centrifugal pumps may be utilized to generate blood flow within the extracorporeal perfusion circuit.

In some extracorporeal perfusion systems, the oxygenator may include a housing having multiple chambers or pathways separated by a semi-permeable membrane, whereby the patient's blood may flow through one chamber or pathway, while an oxygen gas mixture (i.e., sweep gas) flows through another chamber or pathway. The semi-permeable membrane may include multiple microporous hollow fibers, each fiber having a lumen extending therethrough through which the oxygen gas mixture flows. The gas exchange may occur via diffusion of the gases across multiple microporous fibers, whereby oxygen moves from the inside of the hollow fibers into the blood while carbon dioxide diffuses from the blood into the interior of the hollow fibers, where it is swept away by the sweep gas flowing through the fiber. This gas exchange allows for oxygenation of venous blood and removal of carbon dioxide. In some extracorporeal perfusion systems, the oxygenator may include integrated heat exchangers that allow circulating blood to be cooled and/or warmed prior to returning to the patient.

In some instances, it may be desirable to control one or more parameters of blood flow through the various blood pathways of the extracorporeal perfusion system. For example, it may be desirable to control the flowrate of blood within one or more blood pathways within the extracorporeal perfusion system. In some examples, the flowrate of blood within the blood pathways of the extracorporeal perfusion system may be controlled via a combination of clamps, sensors and pumps, one or more of which may be coupled to the various blood pathways of the extracorporeal perfusion system. In some examples, one or more of the clamps, sensors and pumps may be in communication (e.g., wireless, wired communication, or other communication means capable of transmitting signals) with a control unit, whereby the control unit may be configured to operate one or more of the clamps, sensors and pumps in various combinations to control the flow of blood within the blood pathways of the extracorporeal perfusion system.

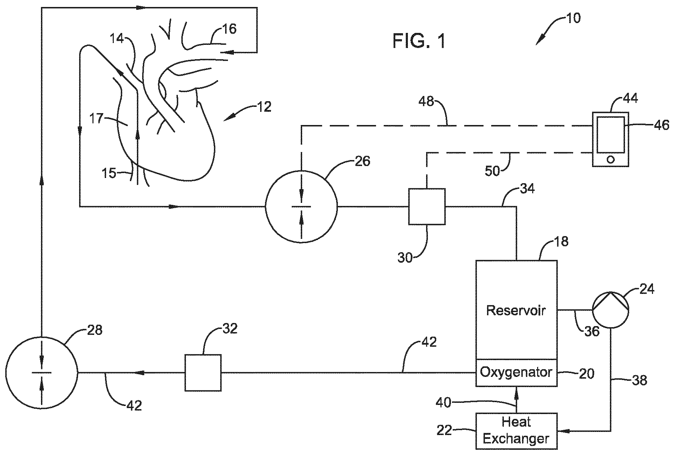

FIG. 1 illustrates an extracorporeal perfusion system 10. The extracorporeal perfusion system 10 may include a blood reservoir 18, an oxygenator 20, a heat exchanger 22, clamps 26, 28, sensors 30, 32, a pump 24 and a control unit 44. Additionally, the extracorporeal perfusion system 10 may include one or more blood pathways extending between various components of the extracorporeal perfusion system 10.

The blood reservoir 18 of the extracorporeal perfusion system 10 may be designed to hold blood which is gravity fed from a patient, such as the patient's superior vena cava (SVC) 14 and inferior vena cava (IVC) 15 or, alternatively, from a single cannula placed in the patient's right atrium 17. Generally, blood from the reservoir 18 may then pass to a blood pump 24 along a blood pathway 36. The pump 24 may then pump the blood along a blood pathway 38 into a heat exchanger 22. After passing through the heat exchanger 22, the blood may pass into an oxygenator 20 along a blood pathway 40. After gas exchange takes place within the semi-permeable membrane of the oxygenator 20, the post-oxygenated blood may return to the arterial system of the patient, such as via a cannula placed in the aorta 16.

In some examples, the blood reservoir 18, the oxygenator 20, and/or the heat exchanger 22 may be coupled together in a variety of configurations. For example, the oxygenator 20 and the heat exchanger 22 may be combined into a single unit. In other examples, the oxygenator 20 and the heat exchanger 22 may be combined into a single unit, while the reservoir may be coupled (e.g., clipped, secured, attached, etc.) to the oxygenator 20. In yet other examples, the blood reservoir 18, the oxygenator 20, and/or the heat exchanger 22 may be separate components within the extracorporeal perfusion system 10.

As discussed herein, the extracorporeal perfusion system 10 shown in FIG. 1 may also include one or more clamps 26, 28 and sensors 30, 32 positioned along various blood pathways (e.g., venous and arterial blood pathways), whereby the clamps 26, 28 and sensors 30, 32, may help regulate the flow of blood through the blood pathways. For example, FIG. 1 illustrates that the extracorporeal perfusion system 10 may include a clamp 26 and sensor 30 positioned along the venous blood pathway 34 and a clamp 28 and sensor 32 positioned along the arterial return blood pathway 42. The arterial return blood pathway 42 may be defined as the blood pathway 42 along which oxygenated blood exiting the oxygenator 20 flows back to the aorta 16 of the patient.

When coupled to the venous blood pathway 34, the clamp 26 may be configured to actuate such that the clamp 26 decreases or increases the cross-sectional area of a component defining the venous blood pathway 34. For example, the venous blood pathway 34 may be formed from a tubing having a wall and a lumen extending therein. The lumen of the tubing may have a cross-sectional area which, along with the velocity of the blood flowing through the tubing, defines the volume of blood which may pass through the tubing over a given time period.

In some examples, the tubing used to define the venous blood pathway 34 may be formed from a polymer tubing (e.g., polyvinyl tubing). For example, the tubing used to define the venous blood pathway 34 may be constructed from polyvinyl chloride (PVC) because it is flexible, compatible with blood, inert, nontoxic, smooth, tough, transparent, resistant to kinking and collapse, and may be heat sterilized.

In other examples, the venous blood pathway 34 may be formed from other structures and materials. Additionally, some alternative materials that may be utilized to form the venous blood pathway 34 may include silicone. For example, the venous blood pathway 34, or portions thereof, may be formed from rigid tubing components having a lumen extending therethrough.

As discussed herein, when positioned along the blood pathway 34, the clamp 26 may be configured to actuate such that the clamp 26 decreases or increases the cross-sectional area of a lumen of a component defining the venous blood pathway 34. In some examples, the clamp 26 may engage tubing defining the blood pathway 34. In these examples, the tubing defining the blood pathway 34 may extend within at least a portion of the clamp 26, whereby actuation of the clamp 26 may either clamp down and restrict the cross-sectional area of the tubing or may release and expand the cross-sectional area of the tubing defining the blood pathway 34. In other words, the clamp 26 may be designed to physically deform the tubing to adjust the cross-sectional area of the lumen (which may, in turn, increase the resistance of the tubing), and therefore, the flowrate of blood through the tubing.

In other examples, the clamp 26 may include a component (e.g., a ball valve, iris, etc.) having an adjustable lumen size and/or restriction designed to adjust the flowrate of blood through the clamp 26. The clamp 26 may be designed such that a first section of tubing (e.g., flexible, semi-rigid, rigid tubing) may be inserted into an inlet of the clamp 26 and a second section of tubing may be inserted into an outlet of the clamp 26. Accordingly, the blood may flow through the first section of tubing into the clamp 26, through a valve located in the clamp 26, and exit the clamp 26 via an outlet of the clamp 26 and into the second section of tubing.

FIG. 1 further illustrates that the extracorporeal perfusion system 10 may include a sensor 30 positioned along the venous blood pathway 34. In some examples the sensor 30 may be fixedly attached to the clamp 26 (e.g., the sensor 30 may be an integrated component of the clamp 26). However, in other examples the sensor 30 may be a separate and distinct component, separated from the clamp 26 and positioned along any portion of the venous blood pathway 34. In some examples, the sensor 30 may be positioned on an inner surface, the outer surface or within a wall of the tubing defining the venous blood pathway 34. In other examples, the sensor 30 may be positioned adjacent to a component (e.g., tubing) defining the blood pathway 34.

The sensor 30 may be a flow sensor configured to sense (e.g., detect, measure, compute, monitor, etc.) the flowrate of blood passing through the blood pathway 34. Additionally, the extracorporeal perfusion system 10 may include additional sensors positioned along the blood pathway 34. For example, the extracorporeal perfusion system 10 may include one or more sensors for monitoring pressures, temperatures, bubbles, oxygen saturation, carbon dioxide content, blood gases, or other blood parameters. Further, in some instances a single sensor may be configured to sense multiple blood parameters including blood flowrate, pressures, temperatures, bubbles, oxygen saturation, carbon dioxide content, blood gases, etc.

It can be appreciated that the clamp 28 may be of similar form and function to the clamp 26 described herein. The clamp 28 may include all the features and operate substantially similar to the clamp 26 described herein. Additionally, it can be appreciated that the sensor 32 may be similar in form and function to the sensor 30 described herein. The sensor 32 may include all the features of and operate substantially similar to the sensor 30 described herein.

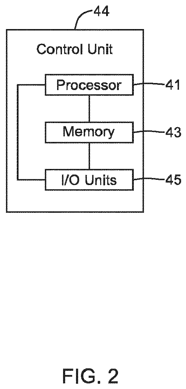

As discussed herein, the extracorporeal perfusion system 10 may also include a control unit 44. The control unit 44 may include a visual display 46 and/or one or more control knob (e.g., buttons). FIG. 2 further illustrates that the control unit 44 may include, among other suitable components, a processor 41, memory 43, and an I/O unit 45.

The processor 41 of the control unit 44 may include a single processor or more than one processor working individually or with one another. The processor 41 may be configured to execute instructions, including instructions that may be loaded into the memory 43 and/or other suitable memory. Example processor components may include, but are not limited to, microprocessors, microcontrollers, multi-core processors, graphical processing units, digital signal processors, application specific integrated circuits (ASICs), field programmable gate arrays (FPGAs), discrete circuitry, and/or other suitable types of data processing devices.

The memory 43 of the control unit 44 may include a single memory component or more than one memory component each working individually or with one another. Example types of memory may include random access memory (RAM), EEPROM, FLASH, suitable volatile storage devices, suitable non-volatile storage devices, persistent memory (e.g., read only memory (ROM), hard drive, Flash memory, optical disc memory, and/or other suitable persistent memory) and/or other suitable types of memory. The memory 43 may be or may include a non-transitory computer readable medium.

The I/O units 45 of the control unit 44 may include a single I/O component or more than one I/O component each working individually or with one another. Example I/O units 45 may be any type of communication port configured to communicate with other components of the building management system. Example types of I/O units 45 may include wired ports, wireless ports, radio frequency (RF) ports, Low-Energy Bluetooth ports, Bluetooth ports, Near-Field Communication (NFC) ports, HDMI ports, Wi-Fi ports, Ethernet ports, VGA ports, serial ports, parallel ports, component video ports, S-video ports, composite audio/video ports, DVI ports, USB ports, optical ports, and/or other suitable ports.

Additionally, the control unit 44 may be in communication with various components of the extracorporeal perfusion system 10. For example, FIG. 1 illustrates that the control unit 44 may be in communication (e.g., wireless, wired communication, or other communication means capable of transmitting signals) with the clamp 26 and the sensor 30, both of which may be positioned along the venous blood pathway 34. In some examples, the control unit 44 may be integrated into a console or work station of the extracorporeal perfusion system 10. In other examples, the control unit 44 may be integrated directly into a heart-lung machine. The control unit 44 may be in direct or indirect communication with a console, work station and/or a heart-lung machine.

Further, the clamp 26, the sensor 30 (e.g., flow sensor, pressure sensor, etc.) and the control unit 44 may together form a closed-loop system capable of automatically or manually regulating the flowrate of blood within the venous blood pathway 34. In some examples, the flowrate within the blood pathway may be from 0-8 liters/min at a pressure between -200 mmHg and +800 mmHg. For example, the sensor 30 may be configured to sense a first parameter (e.g., flowrate, pressure, etc.) of blood passing through the blood pathway 34. Additionally, the sensor 30 may be configured to transmit a signal corresponding to the sensed parameter (e.g., flowrate, pressure, etc.) to the control unit 44. Further, the control unit 44 may be configured to receive the signal (corresponding to the sensed flowrate and/or pressure of the blood within the blood pathway 34) transmitted by the sensor 30. The control unit 44 may be configured to compare the signal received from the sensor 30 to a parameter (e.g., flowrate, pressure, etc.) set point input by a clinician into the control unit 44. After comparing the signal received from the sensor 30, the control unit 44 may transmit a signal to the clamp 26. The clamp 26 may be configured to receive the signal from the control unit 44. After receiving and processing the signal from the control unit 44, the clamp 26 may be automatically actuated to adjust the blood flow (e.g., the flowrate of the blood) through the blood pathway 34 in response to receiving the signal from the control unit 44. In some examples, the clamp 26 may send a signal back to the control unit 44 confirming the position to which the aperture of the clamp 26 has been actuated (e.g., the clamp 26 may send a signal indicating the size of the aperture through which the blood is flowing, such as a percentage that that the clamp 26 is opened). It can be appreciated that a component (e.g., console unit 44, clamp 26, sensor 30, etc.) of the extracorporeal perfusion system 10 may include an algorithm which utilizes the sensed flowrate data from the sensor 30 to calculate the appropriate automatic actuation of the clamp 26 required to meet the clinician's desired blood flowrate within the blood pathway 34. It can be further appreciated that the set point and/or set range of values for the flowrate of blood through the venous blood pathway 34 may be input by a clinician via the control features (e.g., display, dial, button, etc.) 46 of the control unit 44 or other components of the extracorporeal perfusion system 10 (e.g., heart-lung machine). In other words, a clinician may be able to input a set point or a set range of values for blood flowrates in various blood pathways in the system via a touchpad, dial, control knob, etc.

As discussed herein, after receiving and processing the signal from the control unit 44, the clamp 26 may be automatically actuated to adjust the blood flowrate through the blood pathway 34 in response to receiving the signal from the control unit 44. In some examples, a component (e.g., console unit 44, clamp 26, sensor 30, etc.) of the extracorporeal perfusion system 10 may include an algorithm which utilizes sensed blood pressure data from the sensor 30 to calculate the appropriate automatic actuation of the clamp 26 required to meet the clinician's desired blood pressure within the blood pathway 34. It can be appreciated that the set point or set range of values for the pressure of blood through the venous blood pathway 34 may be input by a clinician via the display 46 of the control unit 44. In other words, a clinician may be able to input a set point or set range of values for blood pressure in various blood pathways in the system via a touchpad, dial, control knob, etc.

As described herein, the control unit 44 (and all control units described herein) may permit a user (e.g., perfusionist, clinician, etc.) to input pre-defined values or a pre-defined range of values for the flowrate of blood within the venous blood pathway 34 (independent of the arterial blood pathway 42), the arterial blood pathway 42 (independent of the venous blood pathway 34) or both the venous blood pathway 34 and the arterial blood pathway 42. In some examples, the flowrate of blood in the venous blood pathway 34 may be regulated by the clamp 26 positioned in the venous blood pathway 34. The actuation of the clamp 26 may control the flowrate of blood in the venous blood pathway 34, whereby the actuation of the clamp 26 is determined by a flowrate of venous blood as measured by a flow sensor 30. Additionally, the actuation of the clamp 26 may control the flowrate of blood in the venous blood pathway 34, whereby the actuation of the clamp 26 is determined by a pressure of the venous blood as measured by a pressure sensor 30 (e.g., the pressure of the blood in the venous blood pathway 34 may be correlated to the flowrate of blood within the venous blood pathway 34).

Further, as will be described herein, actuation of the clamp 26 may control the volume of blood maintained within the reservoir 18. A level sensor (e.g., volume sensor, mass sensor, etc.) may be positioned within the reservoir 18, whereby the level sensor may measure the level (e.g., volume) of blood within the reservoir 18. The level sensor may communicate with the control unit 44 to open/close the clamp 26 in response to the level of blood sensed by the level sensor in the reservoir 18. It can be appreciated that a clinician may input a pre-defined set point or range of values of the desired level of blood to be maintained in the reservoir 18.

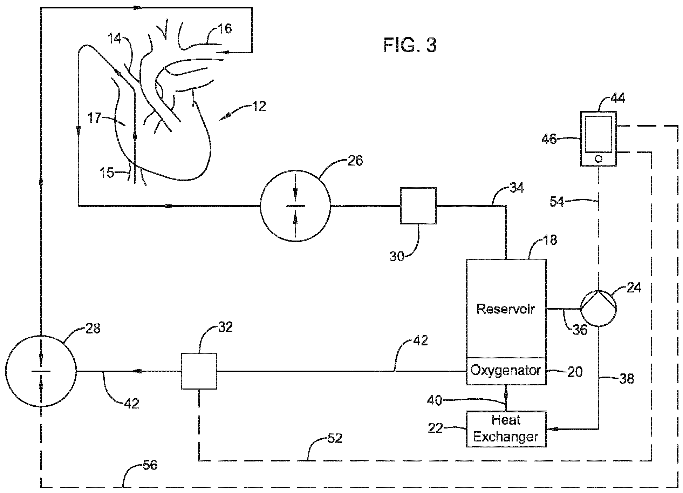

Further, in some examples, a centrifugal pump may be positioned within the venous blood pathway 34, whereby the centrifugal pump may operate in combination with the clamp 26, the sensor 30 (e.g., flow sensor, pressure sensor) and/or a level sensor (positioned in the reservoir 18) to control the flowrate of blood with the venous blood pathway 34 and/or the level of blood within the reservoir 18. The components of the extracorporeal perfusion system 10 described herein may be interconnected in a variety of configurations to monitor and regulate blood flow through the various blood pathways of the extracorporeal perfusion system 10. For example, FIG. 3 illustrates the extracorporeal perfusion system 10 in which the control unit 44 is in communication (e.g., wired, wireless communication, or other communication means capable of transmitting signals) with the clamp 28 (positioned along the arterial return blood pathway 42), the sensor 32 (positioned along the arterial return blood pathway 42) and the blood pump 24.

Similar to that described herein with respect to FIG. 1, the clamp 28, the sensor 32 (e.g., flow sensor) and the pump 24 may form a closed-loop system capable of regulating the flowrate and/or pressure of blood within the arterial blood return pathway 34. For example, as illustrated by the dashed line 52, the sensor 32 may monitor and communicate the flowrate of blood flowing within the arterial blood pathway 42 directly with the control unit 44. Additionally, as illustrated by the dashed line 56, the clamp 28 may communicate directly with the control unit 44. Further, the sensor 32 may be configured to sense a first parameter (e.g., flowrate, pressure, etc.) of blood passing through the blood pathway 42. Additionally, the sensor 32 may be configured to transmit a signal corresponding to the sensed parameter (e.g., flowrate, pressure, etc.) to the control unit 44. Further, the control unit 44 may be configured to receive the signal (corresponding to the sensed flowrate and/or pressure of the blood within the blood pathway 42) transmitted by the sensor 32. The control unit 44 may be configured to compare the signal received from the sensor 32 to a parameter (e.g., flowrate, pressure, etc.) set point input by a clinician into the control unit 44. After comparing the signal received from the sensor 32, the control unit 44 may transmit a signal to the clamp 28, the pump 24 or both the clamp 28 and the pump 24. Both the clamp 28 and the pump 24 may be configured to receive the signal from the control unit 44. After receiving and processing the signal from the control unit 44, the clamp 28 may be automatically actuated to adjust the blood flow (e.g., the flowrate of the blood) through the blood pathway 42 in response to receiving the signal from the control unit 44. In some examples, the clamp 28 may send a signal back to the control unit 44 confirming the position to which the aperture of the clamp 28 has been actuated (e.g., the clamp 28 may send a signal indicating the size of the aperture through which the blood is flowing, such as a percentage that that the clamp 28 is opened). Additionally, after receiving and processing the signal from the control unit 44, the pumping action of the pump 24 (e.g., increasing or decreasing the rotational speed of the pump 24) may be manually or automatically adjusted (e.g., increased or decreased) to adjust the blood flowrate through the blood pathway 42 in response to receiving the signal from the control unit 44. In some examples, the pump 24 may send a signal back to the control unit 44 confirming the adjusted (e.g., increased or decreased) speed of the pump 24. Further, a component (e.g., console unit 44, clamp 26, clamp 28, sensor 30, sensor 32, etc.) of the extracorporeal perfusion system 10 may include an algorithm which utilizes the sensed flowrate and/or pressure data from the sensor 32 to calculate the appropriate automatic actuation of the clamp 28 and the increase/decrease in the pumping action of the pump 24 required to meet the clinician's desired blood flowrate and/or pressure within the arterial return blood pathway 42.

It can be appreciated that in some instances the blood pump 24 may be maintained at a relatively low, steady speed, while the automatic actuation of the clamp 28 may be utilized as the primary mechanism to regulate flow with the arterial return blood pathway 42. It can be further appreciated that the set point for the flowrate of blood through the arterial blood pathway 42 may be input by a clinician via the display 46 of the control unit 44. In other words, a clinician may be able to input a set point for blood flowrates in various blood pathways in the system via a touchpad, dial, control knob, etc. In other instances, after receiving and processing the signal from the control unit 44, the clamp 28 may be automatically actuated to adjust the blood flowrate through the blood pathway 42 in response to receiving the signal from the control unit 44. It can be appreciated that a component (e.g., console unit 44, clamp 26, clamp 28, sensor 30, sensor 32, etc.) of the extracorporeal perfusion system 10 may include an algorithm which utilizes the sensed blood flowrate data from the sensor 32 to calculate the appropriate automatic actuation of the clamp 28 required to meet the clinician's desired blood flowrate within the blood pathway 42. It can be appreciated that the set point for the flowrate of blood through the arterial blood pathway 42 may be input by a clinician via the display 46 of the control unit 44. In other words, a clinician may be able to input a set point for blood flowrate in various blood pathways in the system via a touchpad, dial, control knob, etc.

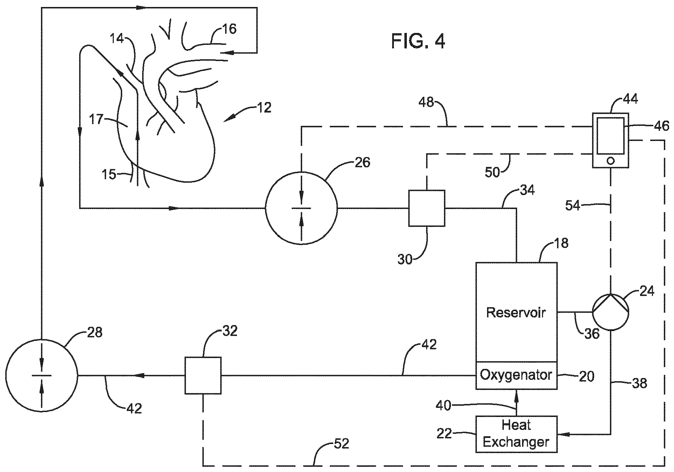

FIG. 4 illustrates the extracorporeal perfusion system 10 in which the control unit 44 is in communication (e.g., wired, wireless communication, or other communication means capable of transmitting signals) with the clamp 26 (positioned along the venous blood pathway 42), the sensor 30 (positioned along the venous blood pathway 42), the sensor 32 (positioned along the arterial return blood pathway 42) and the blood pump 24.

As described herein, the clamp 26, the sensor 30 (e.g., flow sensor) and the control unit 44 may together form a first closed-loop system capable of regulating the flowrate of blood within the venous blood pathway 34. Similarly, as described herein, the sensor 32 (e.g., flow sensor, pressure sensor, etc.) and the pump 24 may form a form a closed-loop system capable of regulating the flowrate of blood within the arterial blood return pathway 42. For example, as illustrated by the dashed line 52, the sensor 32 may monitor and communicate the flowrate of blood flowing within the arterial blood pathway 42 directly with the control unit 44. Additionally, as illustrated by the dashed line 54, the pump 24 may communicate directly with the control unit 44.

Further, it can be appreciated that, in any of the examples disclosed herein, the control unit 44 may be configured to automatically monitor and compare the flowrate of blood within the venous blood pathway 34 to the flowrate of blood within the arterial return blood pathway 42. Further, in some examples, a pre-defined value or a pre-defined range of values for the flowrate of blood within the venous blood pathway 34 and/or the flowrate of blood within the arterial return blood pathway 42 may be input via the control unit 44 by a clinician. Additionally, in some instances it may be beneficial for the flowrate of blood in the venous blood pathway 34 to be substantially equal to the flowrate of blood within the arterial return blood pathway 42. In other examples, it may be beneficial to define a ratio of the flowrate of blood within the arterial blood pathway 42 and the flowrate of blood within the venous blood pathway 34 relative to a pre-defined value or pre-defined range of values of the flowrate of blood within the venous pathway 34, and the control unit 44 may be configured to maintain the defined ratio automatically. In other examples, it may be beneficial to define a ratio of the flowrate of blood within the arterial blood pathway 42 and the flowrate of blood within the venous blood pathway 34 relative to a pre-defined value or pre-defined range of values of the flowrate of blood within the arterial pathway 42, and the control unit 44 may be configured to maintain the defined ratio automatically. In other examples, the desired level of blood in the reservoir 18 may be set at a pre-defined level or a pre-defined range of levels while the flow rate of blood within the venous blood pathway 34 is pre-defined (e.g., the arterial blow flowrate is regulated), and the control unit 44 may be configured to maintain the defined blood level in the reservoir 18 automatically. In yet other examples, the desired level of blood in the reservoir 18 may be set at a pre-defined level or a pre-defined range of levels while the flow rate of blood within the arterial blood pathway 42 is pre-defined (e.g., the venous flow flowrate is regulated), and the control unit 44 may be configured to maintain the defined blood level in the reservoir 18 automatically.

It can be appreciated that the set point for the flowrate of blood through both the venous blood pathway 34 and the arterial blood pathway 42 may each be input by a clinician via the display 46 of the control unit 44. In other examples, the control unit 44 may be configured to equalize the flowrate of blood through both the venous blood pathway 34 and the arterial return blood pathway 42 via a single input control (e.g., the display 46 of the control unit 44 may include a single button to equalize the flowrate of blood through both the venous blood pathway 34 and the arterial return blood pathway 42). Equalizing the flowrate of blood through both the venous blood pathway 34 and the arterial blood pathway 42 may be useful in the weaning phase of surgery.

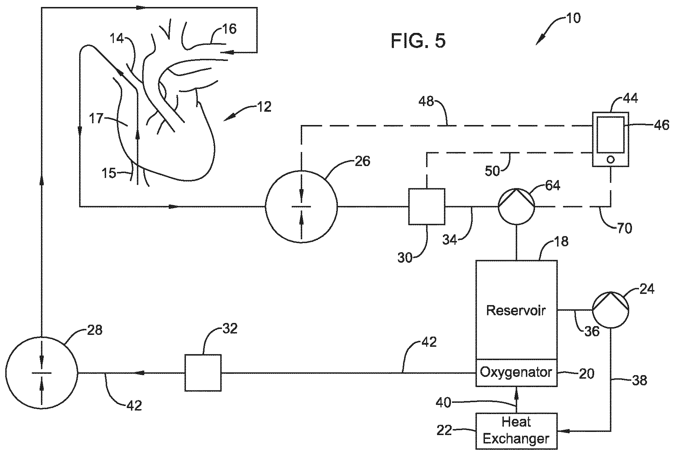

As illustrated in FIG. 5, the extracorporeal perfusion system 10 described herein may include the control unit 44 in communication (e.g., wireless, wired communication, or other communication means capable of transmitting signals) with the clamp 26 and the sensor 30, both of which may be positioned along the venous blood pathway 34. Additionally, as illustrated in FIG. 5, the extracorporeal perfusion system 10 may further include a pump 64 (e.g., roller pump, centrifugal pump, etc.) which may be positioned along the venous blood pathway 34. The pump 64 may be in communication (e.g., wireless, wired communication, or other communication means capable of transmitting signals) with the control unit 44.

In some instances, the gravitational blood flow from a patient to the reservoir 18 may be insufficient to support adequate blood flow through the extracorporeal perfusion circuit. Accordingly, in some instances the pump 64 may be utilized to increase blood flow to the reservoir 18. Like other closed-loop systems described herein, the sensor 30 may be configured to sense a first parameter (e.g., flowrate, pressure, etc.) of gravity-fed blood passing through the venous blood pathway 34. Additionally, the sensor 30 may be configured to transmit a signal corresponding to the sensed parameter (e.g., flowrate, pressure, etc.) to the control unit 44. Further, the control unit 44 may be configured to receive a signal (corresponding to the sensed flowrate of the blood within the blood pathway 34) transmitted by the sensor 30. The control unit 44 may be configured to compare the signal received from the sensor 30 to a parameter (e.g., flowrate, pressure, etc.) set point (e.g., minimum value, maximum value, pre-defined value, relative value, pre-defined range of values, etc.) for the flowrate of gravity-fed blood from a patient to the reservoir 18. After comparing the signal received from the sensor 30, the control unit 44 may automatically transmit a signal to the clamp 26, the pump 64 or both the clamp 26 and the pump 64. Both the clamp 26 and the pump 64 may be configured to receive the signal from the control unit 44. After receiving and processing the signal from the control unit 44, the clamp 26 may be automatically actuated to adjust the blood flow through the blood pathway 34 in response to receiving the signal from the control unit 44. Additionally, after receiving and processing the signal from the control unit 44, the pumping action of the pump 64 may be manually or automatically increased or decreased to adjust the blood flowrate through the blood pathway 34 in response to receiving the signal from the control unit 44. In other words, a component (e.g., console unit 44, clamp 26, sensor 30, etc.) of the extracorporeal perfusion system 10 may include an algorithm which utilizes the sensed parameter (e.g., flowrate and/or pressure data) from the sensor 30 to calculate the appropriate actuation of the clamp 26 and the increase/decrease in the pumping action of the pump 64 required to increase or decrease blood flowrate within the venous return blood pathway 34.

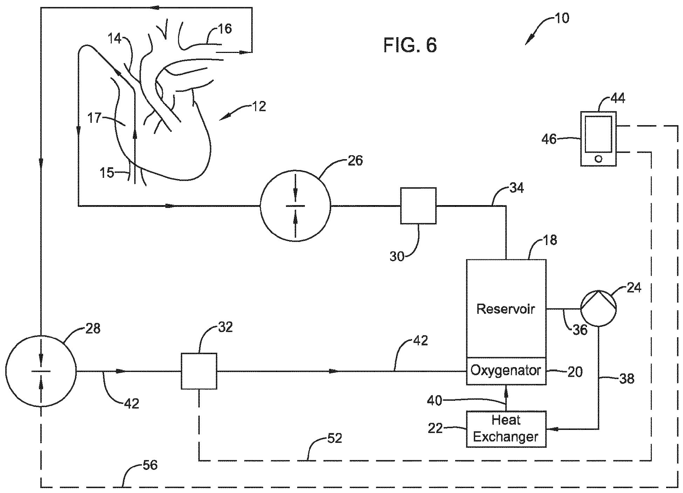

As illustrated in FIG. 6, the extracorporeal perfusion system 10 described herein may include the control unit 44 in communication (e.g., wireless, wired communication, or other communication means capable of transmitting signals) with the clamp 28 and the sensor 32, both of which may be positioned along the arterial return blood pathway 42.

In some instances it may be desirable for blood to flow within the arterial pathway 42 in a retrograde direction. For example, FIG. 6 illustrates blood flowing in a retrograde direction from the aorta 16 toward the oxygenator 20 along the arterial blood pathway 42 (e.g., the arrows along the blood pathway 42 in FIG. 6 illustrate blood flowing from the aorta 16 to the oxygenator 20). Designing the extracorporeal perfusion system 10 to permit retrograde blood flow may be desirable during a RAP (Retrograde Autologous Priming) procedure, in which a patient's own blood is utilized during an initial step to prime the tubing in the extracorporeal perfusion system 10. Priming the tubing in the extracorporeal perfusion system 10 during a RAP procedure replaces the priming fluid (e.g., saline solution) in the tubing of the extracorporeal perfusion system 10 with the patient's own blood. A RAP procedure is utilized to reduce hemodilution and the need for blood transfusions by utilizing the patient's own blood as the initial fill volume for the extracorporeal perfusion system 10.

Additionally, the clamp 28 and the sensor 32 (e.g., flow sensor) may form a closed-loop system capable of regulating the flowrate of retrograde blood flow within the arterial return blood pathway 42. For example, the sensor 32 may be configured to sense a first parameter (e.g., flowrate, pressure, etc.) of retrograde blood flow passing through the arterial return blood pathway 42. Additionally, the sensor 32 may be configured to transmit a signal corresponding to the sensed parameter (e.g., flowrate, pressure, etc.) to the control unit 44. Further, the control unit 44 may be configured to receive the signal (corresponding to the sensed flowrate and/or pressure of the retrograde blood flow within the arterial return blood pathway 42) transmitted by the sensor 32. The control unit 44 may be configured to compare the signal received from the sensor 32 to a parameter (e.g., flowrate, pressure, etc.) set point input by a clinician into the control unit 44. After comparing the signal received from the sensor 32, the control unit 44 may transmit a signal to the clamp 28. The clamp 28 may be configured to receive the signal from the control unit 44. After receiving and processing the signal from the control unit 44, the clamp 28 may be actuated to adjust the retrograde blood flow through the arterial return blood pathway 42 in response to receiving the signal from the control unit 44. In other words, a component (e.g., console unit 44, clamp 28, sensor 30, etc.) of the extracorporeal perfusion system 10 may include an algorithm which utilizes the sensed flowrate and/or pressure data from the sensor 32 to calculate the appropriate actuation of the clamp 28 required to meet the desired retrograde blood flowrate within the arterial blood pathway 42.

It can be appreciated that the clamps 26, 28 described herein may be designed to automatically close to a shutdown condition in response to a shutdown signal from the control unit 44. For example, during a procedure (e.g., a RAP procedure), in response to a signal received from the sensor 32 (e.g., flow sensor, pressure sensor, bubble sensor, level sensor, volume sensor, temperature sensor, carbon dioxide sensor, etc.) or any other sensor positioned along the blood pathway 42, the control unit 44 may send a shutdown signal to the clamp 28, whereby the clamp 28 is configured to automatically actuate very quickly to a shutdown configuration to stop (or significantly reduce) blood flowing through the arterial blood pathway 42. It can be further appreciated that any clamp (e.g., clamps 26, 28) of the extracorporeal perfusion system 10 described herein may be configured to automatically close to a shutdown condition in response to a shutdown signal received from the control unit 44. In some examples, a perfusionist may be able to select which alarms may be relevant for each individual clamp.

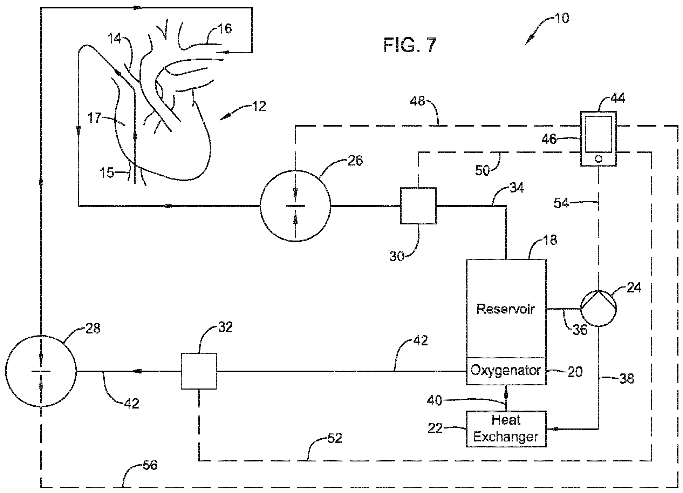

FIG. 7 illustrates the extracorporeal perfusion system 10 in which the control unit 44 may be in communication (e.g., wireless, wired communication, or other communication means capable of transmitting signals) with the clamp 26 (positioned along the venous blood pathway 34), the sensor 30 (positioned along the venous blood pathway 34), the clamp 28 (positioned along the arterial return blood pathway 42), the sensor 32 (positioned along the arterial return blood pathway 42), and the blood pump 24 (e.g., roller pump, centrifugal pump, etc.). Similar to other extracorporeal perfusion systems described herein, the control unit 44, the clamp 26, the sensor 30, the clamp 28, the sensor 32 and the pump 24 may together form one or more closed-loop systems capable of regulating the flowrate of blood within the venous blood pathway 34, the flowrate of blood within the arterial blood return pathway 42 or the flowrate of blood within any other pathway within the extracorporeal perfusion system 10.

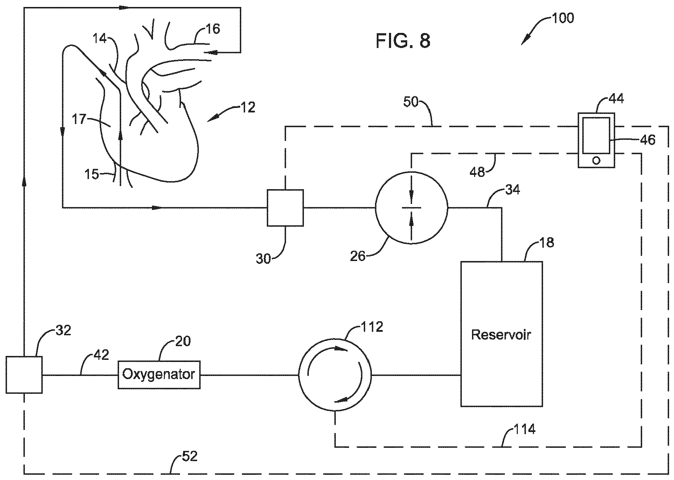

FIG. 8 illustrates another example extracorporeal perfusion system 100. Similar to other perfusion systems described herein, the components of the extracorporeal perfusion system 100 described herein may be interconnected in a variety of configurations to monitor and regulate blood flowrate through the various blood pathways of the extracorporeal perfusion system 100.

For example, FIG. 8 illustrates the extracorporeal perfusion system 100 in which the control unit 44 is in communication (e.g., wired, wireless communication, or other communication means capable of transmitting signals) with the clamp 26 (positioned along the venous blood pathway 34), the sensor 30 (positioned along the venous return pathway 34), the sensor 32 (positioned along the arterial return blood pathway 42) and a pump 112 (e.g., a roller pump, a centrifugal pump, etc. positioned along the arterial return blood pathway 42).

As described herein, the clamp 26, the sensor 30 (e.g., flow sensor), and the control unit 44 may together form a closed-loop system capable of regulating the flowrate of blood within the venous blood pathway 34. Similarly, as described herein, the sensor 32 (e.g., flow sensor, pressure sensor, etc.), the pump 112 and the control unit 44 may together form a closed-loop system capable of regulating the flowrate of blood within the arterial return blood pathway 42. In other examples, any combination of the clamp 26, the sensor 30 (e.g., flow sensor), the sensor 32, the pump 112 and the control unit 44 may together form a closed-loop system capable of regulating the flowrate of blood within the venous blood pathway 34 or the arterial return blood pathway. In some examples, the control unit 44 and/or the pump 112 may include a control panel that permits a user to adjust the speed of the pump 112 in response to the flowrate of blood sensed by the sensor 32 and/or the sensor 30.

It can be appreciated that, in some examples, the control unit 44 may be configured to receive a signal from the pump 112 indicating the speed of the pump 112 and/or a signal from the sensor 32 indicating the flowrate of blood within the arterial return blood pathway 42. Additionally, the control unit 44 may be configured to incrementally open or close the clamp 26 in response to the signals received from the pump 112, the sensor 32 and/or the sensor 30. It can be appreciated that adjusting the clamp 26 may adjust the flowrate of blood along the venous blood pathway 34.

Further, it can be appreciated that the control unit 44 may be configured to automatically monitor and compare the flowrate of blood within the venous blood pathway 34 to the flowrate of blood within the arterial return blood pathway 42. In some instances it may be beneficial to monitor the ratio of the flowrate of blood in the venous blood pathway 34 to the flowrate of blood within the arterial return blood pathway 42. It can be appreciated that the ratio of the flowrate of blood through both the venous blood pathway 34 and the arterial return blood pathway 42 may be input by a clinician via the display 46 of the control unit 44. In other examples, the control unit 44 may be configured to automatically monitor the ratio of the flowrate of blood through both the venous blood pathway 34 and the arterial blood pathway 42. Further, the control unit 44 may be configured to adjust the flowrate of blood within the venous blood pathway 34 and/or the flowrate of blood through the arterial return blood pathway 42 to maintain the ratio of the flowrate of blood through both the venous blood pathway 34 and the arterial return blood pathway 42 within a given range, or to maintain the ratio of the flowrate of blood through both the venous blood pathway 34 and the arterial blood pathway 42 below a maximum threshold value, or to maintain the ratio of the flowrate of blood through both the venous blood pathway 34 and the arterial blood pathway 42 above a minimum threshold value.

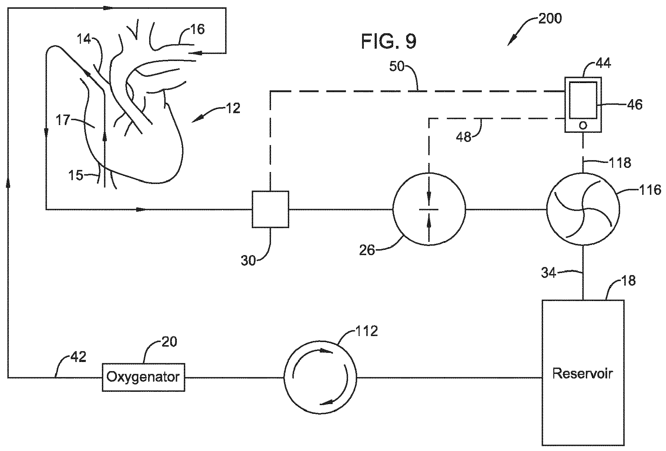

FIG. 9 illustrates another example extracorporeal perfusion system 200. Similar to other perfusion systems described herein, the components of the extracorporeal perfusion system 200 described herein may be interconnected in a variety of configurations to monitor and regulate blood flow through the various blood pathways of the extracorporeal perfusion system 200.

For example, FIG. 9 illustrates the extracorporeal perfusion system 200 in which the control unit 44 is in communication (e.g., wired, wireless communication, or other communication means capable of transmitting signals) with the clamp 26 (positioned along the venous blood pathway 34), the sensor 30 (positioned along the venous return pathway 34) and a pump 116 (e.g., a centrifugal pump, a roller pump, etc.). Similar to other systems described herein, the clamp 26, the sensor 30 (e.g., flow sensor) and the control unit 44 may together form a first closed-loop system capable of regulating the flowrate of blood within the venous blood pathway 34. Similar to that discussed herein with respect to FIG. 5, the gravitational blood flow from a patient to the reservoir 18 may be insufficient to support adequate blood flow through the extracorporeal perfusion circuit. Accordingly, in some instances the pump 116 may be utilized to increase blood flow to the reservoir 18. Like other closed-loop systems described herein, the sensor 30 may be configured to sense a first parameter (e.g., flowrate, pressure, etc.) of gravity-fed blood passing through the blood pathway 34. Additionally, the sensor 30 may be configured to transmit a signal corresponding to the sensed parameter (e.g., flowrate, pressure, etc.) to the control unit 44. Further, the control unit 44 may be configured to receive the signal (corresponding to the sensed flowrate of the blood within the blood pathway 34) transmitted by the sensor 30. The control unit 44 may be configured to compare the signal received from the sensor 30 to a parameter (e.g., flowrate, pressure, etc.) set point corresponding to a minimum threshold for the flowrate of gravity-fed blood from a patient to the reservoir 18. After comparing the signal received from the sensor 30, the control unit 44 may automatically transmit a signal to the clamp 26, the pump 116 or both the clamp 26 and the pump 116. Both the clamp 26 and the pump 116 may be configured to receive the signal from the control unit 44. After receiving and processing the signal from the control unit 44, the clamp 26 may be automatically actuated to adjust the blood flow through the blood pathway 34 in response to receiving the signal from the control unit 44. Additionally, after receiving and processing the signal from the control unit 44, the pumping action of the pump 116 may be manually or automatically increased or decreased to adjust the blood flowrate through the venous blood pathway 34 in response to receiving the signal from the control unit 44. In other words, a component (e.g., console unit 44, clamp 26, sensor 30, etc.) of the extracorporeal perfusion system 10 may include an algorithm which utilizes the sensed flowrate and/or pressure data from the sensor 30 to calculate the appropriate actuation of the clamp 26 and the required increase/decrease in the pumping action of the pump 116 to increase or decrease the blood flowrate within the venous return blood pathway 34.

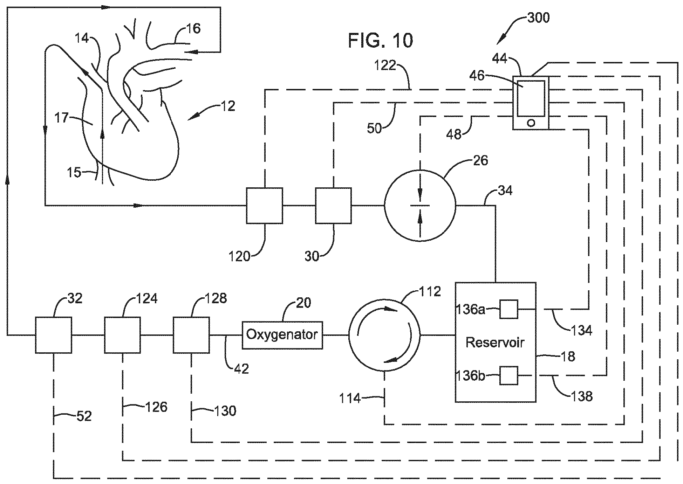

FIG. 10 illustrates another example extracorporeal perfusion system 300. Similar to other perfusion systems described herein, the components of the extracorporeal perfusion system 300 described herein may be interconnected in a variety of configurations to monitor and regulate blood flow through the various blood pathways of the extracorporeal perfusion system 300.

For example, FIG. 10 illustrates the extracorporeal perfusion system 300 in which the control unit 44 is in communication (e.g., wired, wireless communication, or other communication means capable of transmitting signals) with the clamp 26 (positioned along the venous blood pathway 34), the sensor 30 (e.g., positioned along the venous return pathway 34) and a sensor 120 (e.g., positioned along the venous return pathway 34). In some examples the sensors 30, 120 may be fixedly attached to the clamp 26 (e.g., the sensors 30, 120 may be integrated components of the clamp 26). However, in other examples the sensors 30, 120 may be separate and distinct components, separated from the clamp 26 and positioned along any portion of the venous blood pathway 34. In some examples, the sensors 30, 120 may be positioned on an inner surface, the outer surface or within a wall of the tubing defining the venous blood pathway 34. In other examples, the sensors 30, 120 may be positioned adjacent to a component (e.g., tubing) defining the blood pathway 34.

The sensor 30 may be a flow sensor configured to sense (e.g., detect, measure, compute, monitor, etc.) the flowrate of blood passing through the blood pathway 34. Additionally, the sensor 120 may include a pressure sensor configured to sense (e.g., detect, measure, compute, monitor, etc.) the pressure of blood passing through the blood pathway 34. Further, in some instances a single sensor (e.g., the sensor 30 and/or the sensor 120) may be configured to sense multiple blood parameters including blood flowrate, pressures, temperatures, oxygen saturation, bubbles, carbon dioxide content, blood gases, etc.

Additionally, the extracorporeal perfusion system 300 may include multiple components positioned along the arterial return blood pathway 42. For example, FIG. 10 illustrates that the control unit 44 may be in communication (e.g., wired, wireless communication, or other communication means capable of transmitting signals) with the pump 112 (positioned along the arterial return blood pathway 42), the sensor 32 (e.g., positioned along the arterial return blood pathway 42), a sensor 124 (e.g., positioned along the arterial return blood pathway 42) and a sensor 128 (e.g., positioned along the arterial return blood pathway 42).

The sensor 32 may be a flow sensor configured to sense (e.g., detect, measure, compute, monitor, etc.) the flowrate of blood passing through the blood pathway 42. Additionally, the sensor 124 may be a pressure sensor configured to sense (e.g., detect, measure, compute, monitor, etc.) the pressure of blood passing through the blood pathway 42. Additionally, the sensor 128 may be a bubble sensor configured to sense (e.g., detect, measure, compute, monitor, etc.) the presence of bubbles within blood passing through the blood pathway 42. In some instances a single sensor (e.g., the sensor 32, the sensor 124 and/or the sensor 128) may be configured to sense multiple blood parameters including blood flowrate, pressures, temperatures, oxygen saturation, bubble detection, carbon dioxide content, blood gases, etc.

FIG. 10 further illustrates that the extracorporeal perfusion system 300 may further include an oxygenator 20 positioned between the pump 112 and one or more of the sensor 32, the sensor 124 and the sensor 128. It can be appreciated that the pump 112 (e.g., roller pump, centrifugal pump, etc.) may be configured to draw blood from the reservoir 18 and pump the blood along the arterial return blood pathway 42 toward the patient.

FIG. 10 further illustrates that the reservoir 18 may include a first level sensor 136a and a second level sensor 136b. As illustrated by the dashed line 134, the first level sensor 136a may be in communication (e.g., wired, wireless communication, or other communication means capable of transmitting signals) with the control unit 44. Similarly, as illustrated by the dashed line 138, the second level sensor 136b may be in communication (e.g., wired, wireless communication, or other communication means capable of transmitting signals) with the control unit 44.

Similar to other closed-loop systems described herein, the clamp 26, the sensor 30 (e.g., flow sensor), the sensor 120 (e.g., pressure sensor), the sensor 136a, the sensor 136b, and the control unit 44 may together form a closed-loop system capable of automatically or manually regulating the flowrate of blood within the venous blood pathway 34. For example, as illustrated by the dashed line 50, the sensor 30 may monitor and communicate the flowrate of blood flowing within the venous blood pathway 34 directly with the control unit 44. Additionally, as illustrated by the dashed line 122, the sensor 120 may monitor and communicate the pressure of blood flowing within the venous blood pathway 34 directly with the control unit 44. Based on the information sensed by the sensors 30, 120, the control unit 44 may communicate directly with the clamp 26 to increase or decrease the volume of blood flowing through the clamp 26.

Additionally, similar to other closed-loop systems described herein, the pump 112 (e.g., roller pump), the sensor 32 (e.g., flow sensor), the sensor 124 (e.g., pressure sensor), the sensor 128 (e.g., bubble sensor) and the control unit 44 may together form a closed-loop system capable of automatically or manually regulating the flowrate of blood within the arterial blood pathway 42. For example, as illustrated by the dashed line 52, the flow sensor 32 may monitor and communicate the flowrate of blood flowing within the arterial blood pathway 42 directly with the control unit 44. Additionally, as illustrated by the dashed line 126, the pressure sensor 124 may monitor and communicate the pressure of blood flowing within the arterial blood pathway 42 directly with the control unit 44. Further, as illustrated by the dashed line 130, the bubble sensor 128 may monitor and communicate the presence of bubbles within blood flowing within the arterial blood pathway 42 directly with the control unit 44. Based on the information sensed by the sensors 30, 120, 128, the control unit 44 may communicate directly with the pump 112 to increase or decrease the flowrate of blood flowing within the arterial blood pathway 42. It can be appreciated that speeding up or slowing down the pump 112 may increase or decrease the flowrate of blood flowing within the arterial blood pathway 42.

As described herein, the reservoir 18 may include a first level sensor 136a and a second level sensor 136b. The first level sensor 136a may be designed to sense a maximum level (e.g., a maximum threshold) of blood present in the reservoir 18. Additionally, the second level sensor 136b may be designed to sense a minimum level (e.g., a minimum threshold) of blood present in the reservoir 18.

In some examples, the first level sensor 136a and a second level sensor 136b may be utilized as input sensors for the clamp 26. For example, if the flowrate of blood within the venous blood pathway 34 exceeds the flowrate of blood within the arterial return blood pathway 42, the volume of blood within the reservoir 18 may increase over a time period. The volume of blood within the reservoir 18 may continue to increase to a maximum allowed limit, which may be sensed by the first level sensor 136a. The first level sensor 136a may sense that the blood has reached the maximum allowed threshold, whereby the first level sensor 136a may send a signal to the control unit 44. The control unit 44, after receiving the signal from the first level sensor 136a may send a signal to the clamp 26, whereby the clamp 26 may be adjusted, e.g., fully or partially close to restrict the volume of blood flowing therethrough to reduce the amount of blood flowing into the reservoir 18.

Additionally, if the flowrate of blood within the arterial return blood pathway 42 exceeds the flowrate of blood within the venous blood pathway 34, the volume of blood within the reservoir 18 may decrease over a time period. The volume of blood within the reservoir 18 may continue to decrease to a minimum allowed limit, which may be sensed by the second level sensor 136b. The second level sensor 136b may sense that the blood has reached the minimum allowed threshold, whereby the second level sensor 136b may send a signal to the control unit 44. The control unit 44, after receiving the signal from the second level sensor 136b may send a signal to the clamp 26, whereby the clamp 26 may be adjusted, e.g., fully or partially open to increase the amount of blood flowing into the reservoir 18.

Additionally, in some examples, the control unit 44 may be configured to automatically fully or partially close the clamp 26 in response to the pressure of the blood passing through the arterial blood pathway 42 sensed by the sensor 124 (e.g., pressure sensor). Further, in other examples, the control unit 44 may be configured to automatically fully or partially close the clamp 26 and also stop the pump 112 in parallel with the full or partial closing of the clamp 26 in response to the pressure of the blood passing through the arterial return blood pathway 42 sensed by the sensor 124.

Further, in some examples, the control unit 44 may be configured to automatically fully or partially close the clamp 26 in response to the concentration of bubbles present in the blood passing through the arterial return blood pathway 42 sensed by the sensor 128 (e.g., bubble sensor). Further, in other examples, the control unit 44 may be configured to automatically fully or partially close the clamp 26 and also automatically stop the pump 112 in parallel with the full or partial closing of the clamp 26 in response to the concentration of bubbles present in the blood passing through the arterial return blood pathway 42 sensed by the sensor 128.

In some instances, the extracorporeal perfusion system 300 may further include more than one control unit 44. For example, the extracorporeal perfusion system 300 may include two or more control units 44 which together control the flowrate of blood within the venous blood pathway 34 and/or the arterial return blood pathway 42. It can be appreciated that multiple control units 44 may be able utilized to coordinate with one or more components (e.g., the sensors 30, 32, 120, 124, 128, 136a, 136b, clamp 26, pump 112) to control the flowrate of blood with the venous blood pathway 34 and/or the flowrate of blood within the arterial blood pathway 42.

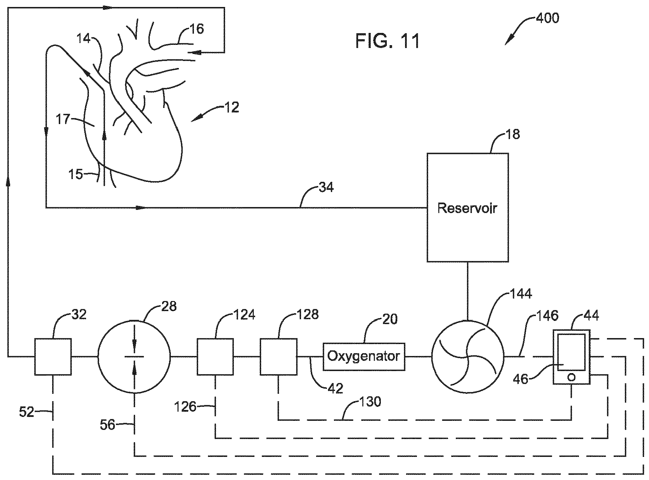

FIG. 11 illustrates another example extracorporeal perfusion system 400. Similar to other perfusion systems described herein, the components of the extracorporeal perfusion system 400 described herein may be interconnected in a variety of configurations to monitor and regulate blood flow through the various blood pathways of the extracorporeal perfusion system 400.

For example, FIG. 11 illustrates a blood reservoir 18 of the extracorporeal perfusion system 400 which may be designed to hold blood which is gravity fed from a patient along the venous blood pathway 34. FIG. 11 illustrates that blood from the reservoir 18 may then pass to a pump 144 (e.g., centrifugal pump) along an arterial return blood pathway 42. It can be appreciated that the pump 144 (e.g., a centrifugal pump) may be configured to draw blood from the reservoir 18 and pump the blood along the blood pathway 42 into an oxygenator 20 where gas exchange may take place within the semi-permeable membrane of the oxygenator 20. Further, the pump 144 may continue to pump the blood along the arterial return blood pathway 42 toward the patient after the oxygenated blood leaves the oxygenator.

FIG. 11 illustrates that after the gas exchange takes place within the oxygenator 20, the blood may pass through the clamp 28 before returning to the patient along the arterial blood pathway 42. Additionally, FIG. 11 illustrates that the extracorporeal perfusion system 400 may include one or more additional components which may be interconnected in a variety of configurations to monitor and regulate blood flow through the arterial blood pathway 42 of the extracorporeal perfusion system 400.

For example, FIG. 11 illustrates that the control unit 44 may be in communication (e.g., wired, wireless communication, or other communication means capable of transmitting signals) with the pump 144 (positioned along the arterial return blood pathway 42), the sensor 32 (e.g., positioned along the arterial return blood pathway 42), a sensor 124 (e.g., positioned along the arterial return blood pathway 42), a sensor 128 (e.g., positioned along the arterial return blood pathway 42) and a clamp 28 (positioned along the arterial return blood pathway 42).

The sensor 32 may be a flow sensor configured to sense (e.g., detect, measure, compute, monitor, etc.) the flowrate of blood passing through the arterial return blood pathway 42. Additionally, the sensor 124 may be a pressure sensor configured to sense (e.g., detect, measure, compute, monitor, etc.) the pressure of blood passing through the arterial return blood pathway 42. Additionally, the sensor 128 may be a bubble sensor configured to sense (e.g., detect, measure, compute, monitor, etc.) the presence of bubbles within blood passing through the arterial return blood pathway 42. In some instances a single sensor (e.g., sensor 32, sensor 124 and/or sensor 128) may be configured to sense multiple blood parameters including blood flowrate, pressures, temperatures, oxygen saturation, bubble detection, carbon dioxide content, blood gases, etc.

Additionally, similar to other closed-loop systems described herein, the pump 144 (e.g., centrifugal pump), the sensor 32 (e.g., flow sensor), the sensor 124 (e.g., pressure sensor), the sensor 128 (e.g., bubble sensor), the clamp 28 and the control unit 44 may together form a closed-loop system capable of automatically or manually regulating the flowrate of blood within the arterial blood pathway 42. For example, as illustrated by the dashed line 52, the flow sensor 32 may monitor and communicate the flowrate of blood flowing within the arterial return blood pathway 42 directly with the control unit 44. Additionally, as illustrated by the dashed line 126, the pressure sensor 124 may monitor and communicate the pressure of blood flowing within the arterial return blood pathway 42 directly with the control unit 44. Further, as illustrated by the dashed line 130, the bubble sensor 128 may monitor and communicate the presence of bubbles within blood flowing within the arterial return blood pathway 42 directly with the control unit 44. Based on the information sensed by the sensors 30, 120, 128, the control unit 44 may communicate directly with the pump 144 to increase or decrease the flowrate of blood flowing within the arterial return blood pathway 42. It can be appreciated that speeding up or slowing down the pump 144 may increase or decrease the flowrate of blood flowing within the arterial return blood pathway 42.

Additionally, the control unit 44 may be configured to automatically fully or partially close the clamp 28 in response to pressure of blood passing through the arterial return blood pathway 42 sensed by the sensor 124 (e.g., pressure sensor). Further, in other examples, the control unit 44 may be configured to automatically fully or partially close the clamp 28 and also automatically stop the pump 144 in parallel with fully or partially closing the clamp 28 in response to the pressure of the blood passing through the arterial return blood pathway 42 sensed by the sensor 124.

Further, in some examples, the control unit 44 may be configured to automatically fully or partially close the clamp 28 in response to the concentration of bubbles present in the blood passing through the arterial return blood pathway 42 sensed by the sensor 128 (e.g., bubble sensor). Further, in other examples, the control unit 44 may be configured to automatically fully or partially close the clamp 28 and also automatically stop the pump 144 in parallel with fully or partially closing the clamp 28 in response to the concentration of bubbles present in the blood passing through the arterial return blood pathway 42 sensed by the sensor 128.

In some instances, the pump 144 (e.g., centrifugal pump) may operate at a constant, relatively low speed (e.g., a minimum speed) while adjustment of the clamp 28 may regulate the flowrate of blood within the arterial blood pathway 42. For example, while the pump 144 is operating at a constant minimum speed, the control unit 44 may receive signals sent by the sensor 32 (e.g., flow sensor), the sensor 124 (e.g., pressure sensor) and the sensor 128 (e.g., bubble sensor) relating to the flowrate of blood with the arterial return blood pathway 42. After processing the signals received by the sensor 32 (e.g., flow sensor), the sensor 124 (e.g., pressure sensor) and the sensor 128 (e.g., bubble sensor), the control unit 44 may send a signal to the clamp 28 which may either partially or fully open or close the clamp 28 to adjust the flowrate of blood within the arterial blood pathway 42.

In some examples, the control unit 44 may be configured to of automatically partially or fully open or close the clamp 28 in response to a flowrate sensed within the arterial blood pathway 42 via the sensor 32 (e.g., flow sensor). In some examples, the control unit 44 may be configured to of automatically partially or fully open or close the clamp 28 in response to a pressure sensed within the arterial blood pathway 42 via the sensor 124 (e.g., pressure sensor). In some examples, the control unit 44 may be configured to of automatically partially or fully open or close the clamp 28 in response to the presence of bubbles sensed within the arterial blood pathway 42 via the sensor 128 (e.g., bubble sensor).

As described herein, the flowrate of blood in the arterial blood pathway 42 may be regulated by the clamp 28 positioned in the arterial blood pathway 42. The actuation of the clamp 28 may control the flowrate of blood in the arterial blood pathway 42, whereby the actuation of the clamp 28 is determined by a flowrate of arterial blood as measured by the flow sensor 32. Additionally, the actuation of the clamp 28 may control the flowrate of blood in the arterial blood pathway 42, whereby the actuation of the clamp 28 is determined by a pressure of the blood as measured by a pressure sensor 124 (e.g., the pressure of the blood in the arterial blood pathway 42 may be correlated to the flowrate of blood within the arterial blood pathway 42).

Further, as will be described herein, actuation of the clamp 28 may control the volume of blood maintained within the reservoir 18. A level sensor (e.g., volume sensor, mass sensor, etc.) may be positioned within the reservoir 18, whereby the level sensor may measure the level (e.g., volume) of blood within the reservoir 18. The level sensor may communicate with the control unit 44 to open/close the clamp 28 in response to the level of blood sensed by the level sensor in the reservoir 18. It can be appreciated that a clinician may input a pre-defined set point or range of values of the desired level of blood to be maintained in the reservoir 18.

Further, in some examples, the centrifugal pump 144 positioned within the arterial blood pathway 42 may operate in combination with the clamp 28, the flow sensor 32, the pressure sensor 124 and/or a level sensor (positioned in the reservoir 18) to control the flowrate of blood with the arterial blood pathway 42 and/or the level of blood within the reservoir 18.

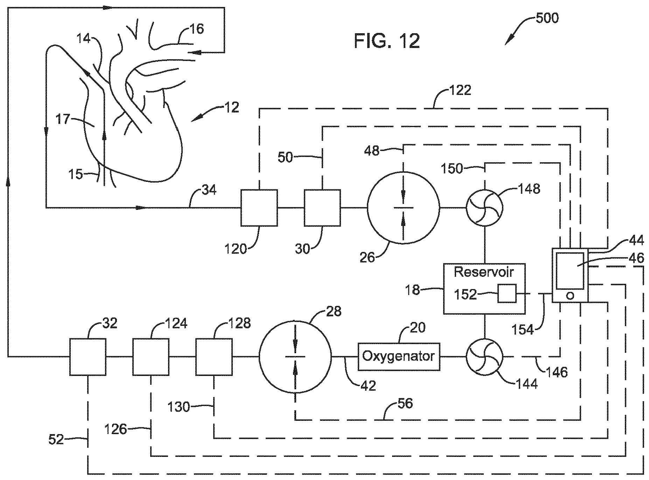

FIG. 12 illustrates another example extracorporeal perfusion system 500. Similar to other perfusion systems described herein, the components of the extracorporeal perfusion system 500 described herein may be interconnected in a variety of configurations to monitor and regulate blood flow through the various blood pathways of the extracorporeal perfusion system 500.

For example, FIG. 12 illustrates the extracorporeal perfusion system 500 in which the control unit 44 is in communication (e.g., wired, wireless communication, or other communication means capable of transmitting signals) with the clamp 26 (positioned along the venous blood pathway 34), the sensor 30 (e.g., positioned along the venous return pathway 34) the sensor 120 (e.g., positioned along the venous return pathway 34) and a pump 148 (e.g., a centrifugal pump positioned along the venous return pathway 34). In some examples the sensors 30, 120 may be fixedly attached to the clamp 26 (e.g., the sensors 30, 120 may be an integrated component of the clamp 26). However, in other examples, the sensors 30, 120 may be separate and distinct components, separated from the clamp 26 and positioned along any portion of the venous blood pathway 34. In some examples, the sensors 30, 120 may be positioned on an inner surface, the outer surface or within a wall of the tubing defining the venous blood pathway 34. In other examples, the sensors 30, 120 may be positioned adjacent to a component (e.g., tubing) defining the blood pathway 34.

The sensor 30 may include a flow sensor configured to sense (e.g., detect, measure, compute, monitor, etc.) the flowrate of blood passing through the blood pathway 34. Additionally, the sensor 120 may include a pressure sensor configured to sense (e.g., detect, measure, compute, monitor, etc.) the pressure of blood passing through the blood pathway 34. Further, in some instances a single sensor (e.g., sensor 30 and/or sensor 120) may be configured to sense multiple blood parameters including blood flowrate, pressures, temperatures, oxygen saturation, carbon dioxide content, bubble detection, blood gases, etc.

Additionally, the extracorporeal perfusion system 500 may include multiple components positioned along the arterial blood pathway 42. For example, FIG. 12 illustrates that the control unit 44 may be in communication (e.g., wired, wireless communication, or other communication means capable of transmitting signals) with the pump 144 (e.g., a centrifugal pump positioned along the arterial blood pathway 42), the sensor 32 (e.g., positioned along the arterial blood pathway 42), a sensor 124 (e.g., positioned along the arterial blood pathway 42), a sensor 128 (e.g., positioned along the arterial blood pathway 42) and a clamp 28 (positioned along the arterial blood pathway 42).

The sensor 32 may be a flow sensor configured to sense (e.g., detect, measure, compute, monitor, etc.) the flowrate of blood passing through the blood pathway 42. Additionally, the sensor 124 may be a pressure sensor configured to sense (e.g., detect, measure, compute, monitor, etc.) the pressure of blood passing through the blood pathway 42. Additionally, the sensor 128 may be a bubble sensor configured to sense (e.g., detect, measure, compute, monitor, etc.) the presence of bubbles within blood passing through the blood pathway 42. In some instances a single sensor (e.g., sensor 32, sensor 124 and/or sensor 128) may be configured to sense multiple blood parameters including blood flowrate, pressures, temperatures, oxygen saturation, bubble detection, carbon dioxide content, blood gases, etc.

FIG. 12 further illustrates that the extracorporeal perfusion system 500 may further include an oxygenator 20 positioned between the pump 144 and one or more of the sensor 32, the sensor 124, the sensor 128 and the clamp 28. It can be appreciated that the pump 144 (e.g., a centrifugal pump) may be configured to draw blood from the reservoir 18 and pump the blood along the arterial blood pathway 42 toward the patient.

FIG. 12 further illustrates that the reservoir 18 may include a level sensor 152. As illustrated by the dashed line 154, the level sensor 152 may be in communication (e.g., wired, wireless communication, or other communication means capable of transmitting signals) with the control unit 44.

Similar to other closed-loop systems described herein, the clamp 26, the sensor 30 (e.g., flow sensor), the sensor 120 (e.g., pressure sensor), the sensor 152, the pump 148 and the control unit 44 may together form a closed-loop system capable of automatically or manually regulating the flowrate of blood within the venous blood pathway 34. For example, as illustrated by the dashed line 50, the flow sensor 30 may monitor and communicate the flowrate of blood flowing within the venous blood pathway 34 directly with the control unit 44. Additionally, as illustrated by the dashed line 122, the pressure sensor 120 may monitor and communicate the pressure of blood flowing within the venous blood pathway 34 directly with the control unit 44. Based on the information sensed by the sensors 30, 120, the control unit 44 may communicate directly with the clamp 26 and/or the pump 148 (e.g., centrifugal pump) to increase or decrease the volume of blood flowing through the clamp 26.

Additionally, similar to other closed-loop systems described herein, the pump 144 (e.g., centrifugal pump), the sensor 32 (e.g., flow sensor), the sensor 124 (e.g., pressure sensor), the sensor 128 (e.g., bubble sensor), the clamp 28 and the control unit 44 may together form a closed-loop system capable of automatically or manually regulating the flowrate of blood within the arterial blood pathway 42. For example, as illustrated by the dashed line 52, the flow sensor 32 may monitor and communicate the flowrate of blood flowing within the arterial blood pathway 42 directly with the control unit 44. Additionally, as illustrated by the dashed line 126, the pressure sensor 124 may monitor and communicate the pressure of blood flowing within the arterial blood pathway 42 directly with the control unit 44. Further, as illustrated by the dashed line 130, the bubble sensor 128 may monitor and communicate the presence of bubbles within blood flowing within the arterial blood pathway 42 directly with the control unit 44. Based on the information sensed by the sensors 30, 120, 128, the control unit 44 may communicate directly with the pump 144 and/or the clamp 28 to increase or decrease the flowrate of blood flowing within the arterial blood pathway 42. It can be appreciated that speeding up or slowing down the pump 144 may increase or decrease the flowrate of blood flowing within the arterial blood pathway 42. It can be further appreciated the partially or fully opening or closing the clamp 28 may increase or decrease the flowrate of blood flowing within the arterial blood pathway 42.

As described herein, the reservoir 18 may include a level sensor 152. The level sensor 152 may be designed to sense a level (e.g., minimum level, maximum level, pre-set threshold level, etc.) of blood present in the reservoir 18. It can be appreciated that a level of blood in the reservoir 18 sensed by the level sensor 152 may correspond to a minimum and/or maximum volume of blood to be permitted in the reservoir 18. In some instances, the level sensor 152 may sense the level of blood present in the reservoir 18 and communicate with the control unit 44 to determine the current volume of blood in the reservoir 18.

In some examples, the level sensor 152 may be utilized as an input sensor 152 for the clamp 26, the clamp 28, the pump 148 and/or the pump 144. For example, if the flowrate of blood within the arterial blood pathway 42 exceeds the flowrate of blood within the venous blood pathway 34, the volume of blood within the reservoir 18 may decrease over a time period. The volume of blood within the reservoir 18 may continue to decrease to a minimum allowed limit, which may be sensed by the level sensor 152. The level sensor 152 may sense that the blood has reached the minimum allowed threshold, whereby the level sensor 152 may send a signal to the control unit 44. The control unit 44, after receiving the signal from the level sensor 152 may send a signal to the clamp 26, the clamp 28, the pump 148 and/or the pump 144, whereby the clamp 26 may fully or partially open (e.g., allowing the volume of blood flowing therethrough) to increase the amount of blood flowing into the reservoir 18, the clamp 28 may fully or partially close (e.g., restrict the volume of blood flowing therethrough) to reduce the amount of blood flowing out of the reservoir 18, the speed of the pump 148 may increase to control the flowrate of blood flowing within the venous blood pathway 34 and/or the speed of the pump 144 may decrease to control the flowrate of blood flowing within the arterial return blood pathway 42.

In other examples, if the flowrate of blood within the venous blood pathway 34 exceeds the flowrate of blood within the arterial blood pathway 42, the volume of blood within the reservoir 18 may increase over a time period. The volume of blood within the reservoir 18 may continue to increase to a maximum allowed limit, which may be sensed by the level sensor 152. The level sensor 152 may sense that the blood has reached the maximum allowed threshold, whereby the level sensor 152 may send a signal to the control unit 44. The control unit 44, after receiving the signal from the level sensor 152 may send a signal to the clamp 26, the clamp 28, the pump 148 and/or the pump 144, whereby the clamp 26 may fully or partially close (e.g., restricting the volume of blood flowing therethrough) to decrease the amount of blood flowing into the reservoir 18, the clamp 28 may fully or partially open (e.g., allowing the volume of blood flowing therethrough) to increase the amount of blood flowing out of the reservoir 18, the speed of the pump 148 may decrease to control the flowrate of blood flowing within the venous blood pathway 34 and/or the speed of the pump 144 may increase to control the flowrate of blood flowing within the arterial return blood pathway 42.

Additionally, in some examples, the control unit 44 may be configured to automatically fully or partially close the clamp 26 and/or the clamp 28 in response to the pressure of the blood passing through the arterial blood pathway 42 sensed by the sensor 124 (e.g., pressure sensor). Further, in other examples, the control unit 44 may be configured to automatically fully or partially close the clamp 26 and/or the clamp 28 and also automatically stop the pump 148 and/or the pump 144 in parallel with fully or partially closing the clamp 26 and/or the clamp 28 in response to the pressure of blood passing through the arterial return blood pathway 42 sensed by the sensor 124.

Further, in some examples, the control unit 44 may be configured to automatically fully or partially close the clamp 26 and/or the clamp 28 in response to the concentration of bubbles in the blood passing through the arterial blood pathway 42 sensed by the sensor 128 (e.g., bubble sensor). Further, in other examples, the control unit 44 may be configured to automatically fully or partially close the clamp 26 and/or the clamp 28 and also automatically stop the pump 148 and/or the pump 144 in parallel with fully or partially closing the clamp 26 and/or the clamp 28 in response to the concentration of bubbles in the blood passing through the arterial return blood pathway 42 sensed by the sensor 128.

In some instances, the extracorporeal perfusion system 500 may further include more than one control unit 44. For example, the extracorporeal perfusion system 500 may include two or more control units 44 which together control the flowrate of blood within the venous blood pathway 34 and/or the arterial return blood pathway 42. It can be appreciated that multiple control units 44 may be utilized to coordinate with one or more components (e.g., the sensors 30, 32, 120, 124, 128, 152, clamp 26, clamp 28, pump 144, pump 148) to control the flowrate of blood with the venous blood pathway 34 and/or the flowrate of blood within the arterial blood pathway 42. Thus, referents to the control unit 44, as used herein, includes multiple control units collectively incorporated into the extracorporeal perfusion system.

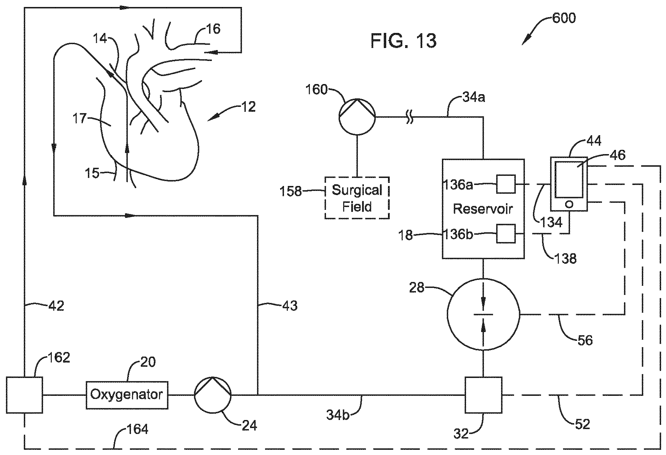

FIG. 13 illustrates another example extracorporeal perfusion system 600. Similar to other perfusion systems described herein, the components of the extracorporeal perfusion system 600 described herein may be interconnected in a variety of configurations to monitor and regulate blood flow through the various blood pathways of the extracorporeal perfusion system 600.

FIG. 13 illustrates that the extracorporeal perfusion system 600 may include a primary blood circuit pathway which includes a pump 24 (e.g., roller pump, centrifugal pump, etc.) designed to draw blood from a patient along a primary venous blood pathway 43. Further, FIG. 13 illustrates that the pump 24 may also pump blood drawn from the patient through an oxygenator 20 and back to the patient along an arterial return blood pathway 42. The pump 24 and the oxygenator 20 may define the primary blood circuit pathway of the extracorporeal perfusion system 600.

FIG. 13 further illustrates that the extracorporeal perfusion system 600 may also include a secondary blood circuit pathway which may be utilized in conjunction with the primary blood circuit pathway of the extracorporeal perfusion system 600. For example, the secondary blood circuit pathway may be utilized to collect blood from the surgical field 158 and then reintroduce the collected blood to the primary blood circuit pathway when the primary blood circuit pathway is not providing the patient with a sufficient amount of blood.

FIG. 13 illustrates that the secondary blood circuit pathway of the extracorporeal perfusion system 600 may include a reservoir 18 which may be designed to hold blood which is collected from the surgical field 158. Blood may be collected from the surgical field 158 via one or more suction pumps 160 and/or one or more vacuum devices. Blood collected from the surgical field 158 may pass to the reservoir 18 along a reservoir inflow pathway 34a. FIG. 13 further illustrates that blood exiting the reservoir 18 may then pass through a clamp 28 along a venous return blood pathway 34b of the secondary blood circuit pathway before combining with blood present in the primary venous blood pathway 43 of the primary blood circuit pathway. It can be appreciated that the pump 24 may be configured to draw blood from the reservoir 18 along the venous blood pathway 43.

FIG. 13 further illustrates that after blood is pulled from the reservoir 18, the blood may pass through a clamp 28 before returning to the patient along the arterial blood pathway 42 after passing through the oxygenator 20. Additionally, FIG. 13 illustrates that the secondary blood circuit pathway may also include a sensor 32 (e.g., flow sensor) positioned between the clamp 28 and the pump 24. Additionally, FIG. 13 illustrates that the control unit 44 may be in communication (e.g., wired, wireless communication, or other communication means capable of transmitting signals) with the sensor 32 (e.g., as shown by the dashed line 52) and the clamp 28 (e.g., as shown by the dashed line 56).

Additionally, FIG. 13 illustrates that after blood passes through the oxygenator 20, the blood may pass by a sensor 162 (e.g., flow sensor, a pressure sensor, a bubble sensor, etc.) before returning to the patient along the arterial blood pathway 42. Additionally, FIG. 13 illustrates that the control unit 44 may be in communication (e.g., wired, wireless communication, or other communication means capable of transmitting signals) with the sensor 162 (e.g., as shown by the dashed line 164) and the clamp 28 (e.g., as shown by the dashed line 56).

FIG. 13 further illustrates that the reservoir 18 may include at least one fluid level sensor, or a plurality of level sensors, such as a first level sensor 136a and a second level sensor 136b. As illustrated by the dashed line 134, the first level sensor 136a may be in communication (e.g., wired, wireless communication, or other communication means capable of transmitting signals) with the control unit 44. Similarly, as illustrated by the dashed line 138, the second level sensor 136b may be in communication (e.g., wired, wireless communication, or other communication means capable of transmitting signals) with the control unit 44.

Additionally, similar to other closed-loop systems described herein, the sensor 32, the sensor 162, the clamp 28, the sensor 136a, the sensor 136b and the control unit 44 may together form a closed-loop system capable of automatically or manually regulating the flowrate of blood within the arterial blood pathway 42. For example, as illustrated by the dashed line 52 and dashed line 164, the sensor 32 and/or the sensor 162 may monitor and communicate the flowrate of blood flowing within the associated blood pathway directly with the control unit 44. Based on the information sensed by the sensor 32 and/or the sensor 162, the control unit 44 may communicate directly with the clamp 28 to incrementally open or close the clamp 28, which may increase or decrease the flowrate of blood flowing within the arterial blood pathway 42.

The first level sensor 136a may be designed to sense a maximum level (e.g., a maximum threshold) of blood to be present in the reservoir 18. Additionally, the second level sensor 136b may be designed to sense a minimum level (e.g., a minimum threshold) of blood to be present in the reservoir 18. In other instances, a single level sensor configured to sense a current level (or volume) of blood in the reservoir 18 may be utilized.

In some examples, the first level sensor 136a and a second level sensor 136b may be utilized as input sensors for the for the clamp 28. For example, if the flowrate of blood within the reservoir inflow pathway 34a exceeds the flowrate of blood within the venous return blood pathway 34b, the volume of blood within the reservoir 18 may increase over a time period. The volume of blood within the reservoir 18 may continue to increase to a maximum allowed limit, which may be sensed by the first level sensor 136a. The first level sensor 136a may sense that the blood has reached the maximum allowed threshold, whereby the first level sensor 136a may send a signal to the control unit 44. The control unit 44, after receiving the signal from the first level sensor 136a may send a signal to the clamp 28, whereby the clamp 28 may automatically fully or partially open (e.g., allowing blood to flow out of the reservoir 18) to reduce the amount of blood in the reservoir 18.

Additionally, if the flowrate of blood within the venous return blood pathway 34b exceeds the flowrate of blood within the reservoir inflow blood pathway 34a, the volume of blood within the reservoir 18 may decrease over a time period. The volume of blood within the reservoir 18 may continue to decrease to a minimum allowed limit, which may be sensed by the second level sensor 136b. The second level sensor 136b may sense that the blood has reached the minimum allowed threshold, whereby the second level sensor 136b may send a signal to the control unit 44. The control unit 44, after receiving the signal from the second level sensor 136b may send a signal to the clamp 28, whereby the clamp 28 may automatically fully or partially close to increase the amount of blood flowing into the reservoir 18 (e.g., increase the volume of blood in the reservoir 18).

In some examples, a level sensor 136a, 136b may be utilized as an input sensor for the clamp 28 and/or the pump 24. For example, the volume of blood within the reservoir 18 may vary during use of the extracorporeal perfusion system 600, which may be sensed by the level sensor 136a, 136b. The level sensor 136a, 136b may send a signal to the control unit 44 indicative of the volume of blood within the reservoir 18. The control unit 44, after receiving the signal from the level sensor 136a, 136b, may send a signal to the clamp 28 and/or the pump 24, whereby the clamp 28 and/or the pump 24 may be adjusted based on the current volume of blood sensed within the reservoir 18.

In some examples, the clamp 28 and/or the pump 24 may be controlled independent of the level sensor 136a, 136b. For example, the volume of blood within the reservoir 18 may vary during use of the extracorporeal perfusion system 600. When desired to return blood collected in the reservoir 18 back to the primary blood circuit pathway and into the patient, the user may use the control unit 44 to send a signal to the clamp 28 and/or the pump 24, whereby the clamp 28 and/or the pump 24 may be adjusted based on the input signal to introduce blood collected in the blood reservoir of the secondary blood circuit pathway into the primary blood circuit pathway.

It can be appreciated that the closed-loop system of the secondary blood circuit pathway described herein with respect to FIG. 13 may be automatically or manually employed to introduce blood collected in the blood reservoir of the secondary blood circuit pathway into the primary blood circuit pathway.

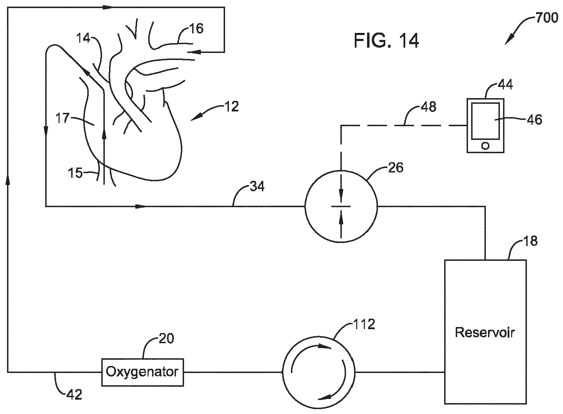

FIG. 14 illustrates another example extracorporeal perfusion system 700. Similar to other perfusion systems described herein, the components of the extracorporeal perfusion system 700 described herein may be interconnected in a variety of configurations to monitor and regulate blood flow through the various blood pathways of the extracorporeal perfusion system 700.

For example, FIG. 14 illustrates the extracorporeal perfusion system 700 in which the control unit 44 is in communication (e.g., wired, wireless communication, or other communication means capable of transmitting signals) with the clamp 26 (positioned along the venous blood pathway 34).

As described herein, the clamp 26 and the control unit 44 may together form a closed-loop system capable of regulating the flowrate of blood within the venous blood pathway 34. Similar to other perfusion systems disclosed herein, FIG. 14 illustrates that the extracorporeal perfusion system 700 may also include a blood reservoir 18 which may be gravity fed from a patient. Further, FIG. 14 illustrates that the extracorporeal perfusion system 700 may also include a pump 112 (e.g., roller pump) and an oxygenator 20 positioned along an arterial return blood pathway 42.

The extracorporeal perfusion system 700 illustrated in FIG. 14 may be utilized, for example, in pediatric cannulation procedures in which relatively low blood flowrates are required to be accurately maintained. In this example, the clamp 26 may include a motor which includes a micro-step controller. The micro-step controller may permit a user to slowly increase or decrease the aperture in the clamp 26, thereby accurately controlling the blood flow within the venous blood pathway 34 and the arterial return blood pathway 42.

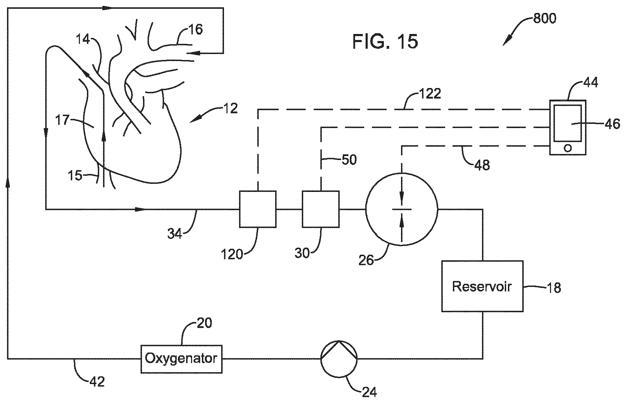

FIG. 15 illustrates another example extracorporeal perfusion system 800. Similar to other perfusion systems described herein, the components of the extracorporeal perfusion system 800 described herein may be interconnected in a variety of configurations to monitor and regulate blood flow through the various blood pathways of the extracorporeal perfusion system 800.

For example, FIG. 15 illustrates the extracorporeal perfusion system 800 in which the control unit 44 is in communication (e.g., wired, wireless communication, or other communication means capable of transmitting signals) with a clamp 26 (positioned along the venous blood pathway 34), a sensor 30 (e.g., positioned along the venous return pathway 34) and a sensor 120 (e.g., positioned along the venous return pathway 34). In some examples the sensors 30, 120 may be fixedly attached to the clamp 26 (e.g., the sensors 30, 120 may be an integrated component of the clamp 26). However, in other examples the sensors 30, 120 may be a separate and distinct component, separate from the clamp 26 and positioned along any portion of the venous blood pathway 34. In some examples, the sensors 30, 120 may be positioned on an inner surface, the outer surface or within a wall of the tubing defining the venous blood pathway 34. In other examples, the sensors 30, 120 may be positioned adjacent to a component (e.g., tubing) defining the blood pathway 34.

The sensor 30 may be a flow sensor configured to sense (e.g., detect, measure, compute, monitor, etc.) the flowrate of blood passing through the blood pathway 34. Additionally, the sensor 120 may include a pressure sensor configured to sense (e.g., detect, measure, compute, monitor, etc.) the pressure of the blood passing through the blood pathway 34. Further, in some instances a single sensor (e.g., the sensor 30 and/or the sensor 120) may be configured to sense multiple blood parameters including blood flowrate, pressures, temperatures, oxygen saturation, carbon dioxide content, bubble detection, blood gases, etc.

Additionally, the extracorporeal perfusion system 800 may include multiple components positioned along the arterial return blood pathway 42. For example, FIG. 15 illustrates that the extracorporeal perfusion system 800 may include a pump 24 (e.g., roller pump, centrifugal pump positioned along the arterial blood pathway 42) and an oxygenator 20 positioned downstream from the pump 24 along the arterial blood pathway 42. It can be appreciated that the pump 24 may be configured to draw blood from the reservoir 18 and pump the blood along the arterial return blood pathway 42 toward the patient.

In some examples, the control unit 44 of the extracorporeal perfusion system 800 may be designed to receive signals from the sensor 120 and/or the sensor 30 and adjust the clamp 26 based on the signals received from the sensor 120 and/or the sensor 30. For example, a user may set the control unit 44 to maintain a desired flowrate within the venous blood pathway 34. In response, the control unit 44 may monitor pressure signals received from the sensor 120 and flowrate signals received from the sensor 30 and incrementally open or close the clamp 26 to maintain the blood flowrate with the venous blood pathway 34 at the desired level.

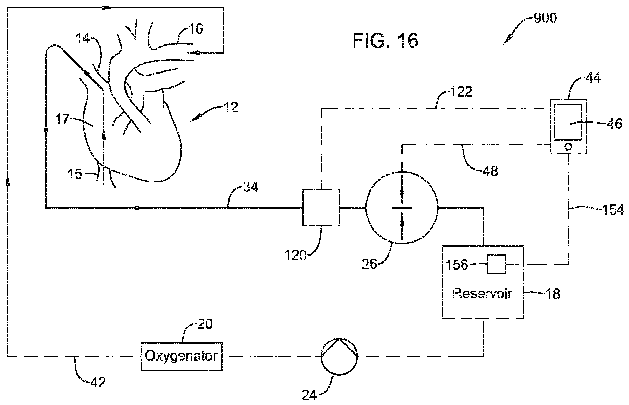

FIG. 16 illustrates another example extracorporeal perfusion system 900. Similar to other perfusion systems described herein, the components of the extracorporeal perfusion system 900 described herein may be interconnected in a variety of configurations to monitor and regulate blood flow through the various blood pathways of the extracorporeal perfusion system 900.

For example, FIG. 16 illustrates the extracorporeal perfusion system 900 in which the control unit 44 is in communication (e.g., wired, wireless communication, or other communication means capable of transmitting signals) with a clamp 26 (positioned along the venous blood pathway 34) and a sensor 120 (e.g., positioned along the venous return pathway 34). In some examples the sensors 30, 120 may be fixedly attached to the clamp 26 (e.g., the sensors 30, 120 may be an integrated component of the clamp 26). However, in other examples the sensors 30, 120 may be a separate and distinct component, separated from the clamp 26 and positioned along any portion of the venous blood pathway 34. In some examples, the sensors 30, 120 may be positioned on an inner surface, the outer surface or within a wall of the tubing defining the venous blood pathway 34. In other examples, the sensors 30, 120 may be positioned adjacent to a component (e.g., tubing) defining the blood pathway 34. FIG. 16 further illustrates that the extracorporeal perfusion system 900 may include a reservoir 18 which may be gravity fed from the patient.

The sensor 120 may include a pressure sensor configured to sense (e.g., detect, measure, compute, monitor, etc.) the pressure of blood passing through the blood pathway 34. Further, in some instances the sensor 120 may be configured to sense multiple blood parameters including blood flowrate, pressures, temperatures, oxygen saturation, bubble detection, carbon dioxide content, blood gases, etc.

Additionally, the extracorporeal perfusion system 900 may include multiple components positioned along the arterial return blood pathway 42. For example, FIG. 15 illustrates that the extracorporeal perfusion system 900 may include a pump 24 (e.g., roller pump, centrifugal pump positioned along the arterial blood pathway 42) and an oxygenator 20 positioned downstream from the pump 24 along the arterial blood pathway 42. It can be appreciated that the pump 24 may be configured to draw blood from a reservoir 18 and pump the blood along the arterial blood pathway 42 toward the patient.

FIG. 16 further illustrates that the reservoir 18 may include a volume sensor 156. As illustrated by the dashed line 154, the volume sensor 156 may be in communication (e.g., wired, wireless communication, or other communication means capable of transmitting signals) with the control unit 44. The volume sensor 156 may be able to sense the volume of blood with the reservoir 18. For example, the volume sensor 156 may be able to sense the volume of blood within the reservoir via sensing the weight and/or pressure of the blood within the reservoir 18. In other examples, the volume sensor 156 may be able to sense the volume of blood within the reservoir via ultrasound.

Similar to other closed-loop systems described herein, the clamp 26, the sensor 120 (e.g., pressure sensor), volume sensor 156 and the control unit 44 may form a closed-loop system capable of automatically or manually regulating the flowrate of blood within the venous blood pathway 34. For example, as illustrated by the dashed line 122, the sensor 120 may monitor and communicate the pressure of blood flowing within the venous blood pathway 34 directly with the control unit 44. Based on the information sensed by the sensor 120, the control unit 44 may communicate directly with the clamp 26 to increase or decrease the volume of blood flowing through the clamp 26.

As described herein, the reservoir 18 may include a volume sensor 156. The volume sensor 156 may be designed to sense a maximum volume of blood present in the reservoir 18. In some examples, the volume sensor 156 may be utilized as an input sensor 156 for the clamp 26. For example, if the flowrate of blood within the venous blood pathway 34 exceeds the flowrate of blood within the arterial blood pathway 42, the volume of blood within the reservoir 18 may increase over a time period. The volume of blood within the reservoir 18 may continue to increase to a maximum allowed limit, which may be sensed by the volume sensor 156. The volume sensor 156 may sense that the blood has reached the maximum allowed threshold, whereby the volume sensor 156 may send a signal to the control unit 44. The control unit 44, after receiving the signal from the volume sensor 156 may send a signal to the clamp 26, whereby the clamp 26 may fully or partially close (e.g., restrict the volume of blood flowing therethrough) to reduce the amount of blood flowing into the reservoir 18. In some examples, a user may be able to set the desired fluid volume on the control unit 44, whereby the closed-loop systems described herein (including the clamp 26, the sensor 120, volume sensor 156 and the control unit 44) controls the clamp 26 to maintain the set volume of blood in the reservoir 18. In other examples, a user may be able to set the reference fluid volume as a blood volume setpoint on the control unit 44, whereby the closed-loop systems described herein (including the clamp 26, the sensor 120, volume sensor 156and the control unit 44) controls the clamp 26 to maintain the set volume of blood in the reservoir 18.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is defined in the language in which the appended claims are expressed.

## Claims

1. An extracorporeal blood treatment system, comprising:
a first clamp coupled to a first blood pathway extending between a patient and a reservoir;
a first sensor positioned along the first blood pathway; and
a control unit in communication with both the first clamp and the first sensor;
wherein the first sensor is configured to sense a first parameter of blood passing through the first blood pathway;
wherein the first sensor is configured to transmit a first signal corresponding to the first parameter to the control unit;
wherein the control unit is configured to receive the first signal and transmit a second signal to the first clamp;
wherein the first clamp is configured to receive the second signal from the control unit;
wherein the first clamp is configured to controllably adjust blood flow through the first pathway in response to receiving the second signal from the control unit.

2. The blood treatment system of claim 1, wherein the first sensor parameter is a first flowrate of blood passing through the first blood pathway.

3. The blood treatment system of claim 2, wherein the first clamp is configured to decrease the first flowrate of blood flowing through the first blood pathway in response to receiving the second signal from the control unit.

4. The blood treatment system of claim 2, wherein the first clamp is configured to increase the first flowrate of blood flowing through the first blood pathway in response to receiving the second signal from the control unit.

5. The blood treatment system of claim 1, wherein the first sensor is directly attached to the first clamp.

6. The blood treatment system of claim 1, wherein the first sensor is spaced away from the first clamp along the first blood pathway.

7. The blood treatment system of claim 1, wherein the first blood pathway defines a venous pathway from the patient to the reservoir.

8. The blood treatment system of claim 1, further comprising a first pump, wherein the first pump is in communication with the control unit.

9. The blood treatment system of claim 8, wherein the control unit is configured to adjust a speed of the first pump based upon the first signal received from the first sensor.

10. The blood treatment system of claim 9, wherein the first clamp is configured to automatically close to a shutdown condition in response to a shutdown signal from the control unit.

11. The blood treatment system of claim 9, wherein the first blood pathway defines an arterial return pathway from the reservoir to the patient.

12. The blood treatment system of claim 8, further comprising a second sensor, wherein the second sensor is positioned along a second blood pathway, and wherein the second sensor is in communication with the control unit.

13. The blood treatment system of claim 12, wherein the second sensor is configured to sense a second parameter of blood passing through the second blood pathway;
wherein the second sensor is configured to transmit a third signal corresponding to the second parameter to the control unit;
wherein the control unit is configured to receive the third signal and transmit a fourth signal to the first pump;
wherein the first pump is configured to adjust blood flow through the second blood pathway in response to receiving the fourth signal from the control unit.

14. The blood treatment system of claim 13, wherein the second parameter is a second flowrate of the blood passing through the second blood pathway.

15. The blood treatment system of claim 14, wherein the first blood pathway defines a venous pathway from the patient to the reservoir, and wherein the second blood pathway defines an arterial return pathway from the reservoir back to the patient.

16. The blood treatment system of claim 15, wherein the control unit is configured to adjust a speed of the first pump based upon the third signal received from the second sensor.

17. The blood treatment system of claim 16, wherein adjusting the speed of the first pump adjusts the second flowrate of the blood passing through the second blood pathway.

18. An extracorporeal blood treatment system, comprising:
a clamp coupled to a venous blood pathway extending between a patient and a reservoir;
a fluid level sensor coupled to the reservoir, the level sensor configured to sense a level of blood in the reservoir; and
a control unit in communication with the clamp and the level sensor;
wherein the level sensor is configured to transmit a first signal to the control unit, wherein the first signal corresponds to a volume of blood in the reservoir;
wherein the control unit is configured to receive the first signal and transmit a second signal to the clamp;
wherein the clamp is configured to receive the second signal from the control unit;
wherein the clamp is configured to controllably adjust blood flow through the venous pathway in response to receiving the second signal from the control unit.

19. The blood treatment system of claim 18, further comprising a first pump positioned in the venous blood pathway, wherein the first pump is in communication with the control unit, and wherein the control unit is configured to adjust a speed of the first pump based upon the first signal received from the level sensor.

20. An extracorporeal blood treatment system, comprising:
a first clamp coupled to a venous blood pathway extending between a patient and a reservoir;
a first sensor positioned along the venous blood pathway;
a second clamp coupled to an arterial blood pathway extending between a patient and a reservoir;
a second sensor positioned along the arterial blood pathway; and
and
a control unit in communication with the first clamp, the first sensor, the second clamp and the second sensor;
wherein the first sensor is configured to transmit a first signal to the control unit, wherein the first signal corresponds to a flowrate of blood in the venous blood pathway;
wherein the second sensor is configured to transmit a second signal to the control unit, wherein the second signal corresponds to a flowrate of blood in the arterial blood pathway;
wherein the control unit is configured to receive the first signal and the second signal;
wherein the control unit is configured to compare the first signal and the second signal; and
wherein the control unit is configured to actuate the first clamp, the second clamp or both the first clamp and the second clamp in response to comparing the first signal and the second signal to controllably adjust blood flow through the venous pathway, the arterial blood pathway or both the venous pathway and the arterial blood pathway.
